# EUROPÄISCHE PATENTSCHRIFT

(11) **EP 3 763 443 B1**
(45) Veröffentlichungstag und Bekanntmachung des Hinweises auf die Patenterteilung: **31.08.2022**
(21) Anmeldenummer: 19186019.6
(22) Anmeldetag: 12.07.2019
(51) Int. Cl.: B01L 9/00, G01N 33/558

(54) **MEHRFACHMAGAZIN FÜR KAPPEN EINES POC-TESTSYSTEMS**
MULTIPLE MAGAZINE FOR CAPS OF A POC TEST SYSTEM
MULTI-MAGASIN POUR COUPELLES D'UN SYSTÈME D'ESSAI POC

(43) Veröffentlichungstag der Anmeldung: 13.01.2021
(73) Patentinhaber: B.R.A.H.M.S GmbH, 16761 Hennigsdorf (DE)
(72) Erfinder: Braun, Michael, 14169 Berlin (DE); Reuter, Patrick, 16761 Hennigsdorf (DE); Ahne, Claudia, 06493 Ballenstedt (DE); Mucha, Andreas, 16540 Hohen Neuendorf (DE)
(74) Vertreter: Hertin und Partner Rechts- und Patentanwälte PartG mbB

(56) Entgegenhaltungen:
- WO-A1-2017/151642
- WO-A2-02/10754
- GB-A- 2 541 375

## Beschreibung

Die Erfindung betrifft in einem Aspekt ein Mehrfachmagazin zum Transport, zur Aufbewahrung und/oder Vorhaltung einer Vielzahl von Kappen für einen POC-Teststreifen, wobei durch gegenüberliegende Zungen und Ausnehmungen an den Innenflächen der Vorder- bzw. Rückwand eine Reihe von zwei oder mehr Fächern zur vertikalen Stapelung der Kappen gebildet werden und wobei das Mehrfachmagazin an einem Kopfende eine schräge Gegenlage aufweist, welche sich über die Breite des Mehrfachmagazins erstreckt. Die Erfindung betrifft zudem Verwendungen des Mehrfachmagazins und einen Kit umfassend ein Mehrfachmagazin sowie eine Vielzahl von Kappen für einen POC-Teststreifen, welche bevorzugt im Mehrfachmagazin gestapelt vorliegen. In einem weiteren Aspekt betrifft die Erfindung ein Verfahren zur Montage eines POC-Testsystems unter Bereitstellung der Kappen mittels des Mehrfachmagazins.

### Hintergrund und Stand der Technik

Die Erfindung betrifft das Gebiet der Fertigung, des Transports und Bereitstellung von Testsystemen insbesondere zur Bestimmung von Biomarkern oder anderen Analyten in kleinen Probenvolumina.

Für eine schnelle medizinische Versorgung haben sogenannte Point-of-Care Tests eine hohe Bedeutung. Der Begriff Point-of-Care-Testing (POCT) bezeichnet in der Medizin in der Regel diagnostische Untersuchungen, die nicht in einem Zentrallabor, sondern unmittelbar in der Nähe des Patienten am sogenannten "point-of-care" durchgeführt werden können. Geeignete POC-Testsysteme zeichnen sich durch geringe Abmessungen, eine leichte Handhabbarkeit und zuverlässige Auslesedaten aus. Zu medizinisch relevanten Analyten zählen z.B. Cholesterin, Glukose, Laktat, Viren, Bakterien, verschiedene Hormone oder andere Biomarker.

Eine Klasse von POC-Testsytemen sind die sogenannten Durchfluss-Testreifen zur Bestimmung von Biomarkern. Beispielsweise sind immunchromatographische Teststreifen bekannt, die über eine Antikörperbindung ein lösliches Biomolekül z.B. ein Hormon oder ein Protein in Blut, Urin oder Speichel mit Hilfe einer Farbreaktion nachweisen. Bekannte POC-Teststreifen sind beispielsweise Schwangerschaftstests oder Blutgerinnungstests, welche mit oder ohne separatem Auslesegerät angeboten werden.

Ferner sind zugehörige Schnelltestverfahren bekannt, wie beispielsweise ein Lateral-Flow-Test (LFT). Bei einem Lateral-Flow-Test wird die zu untersuchende Probe zunächst auf einen Probenbereich auf einem Teststreifen, in der Regel aus porösem Papier oder gesintertem Polymer, aufgetragen. Nach Zugabe eines Laufmittels erfolgt eine Ausbreitung über den Teststreifen aufgrund von Kapillarkräften (Dünnschichtchromatographie). Die Probe wandert mit dem zugegebenen Laufmittel zu einem Bereich, wo sich getrocknete Immunkonjugate mit Salzen und Kohlenhydraten befinden. Die Flüssigkeit löst das Immunkonjugat, wodurch es an die nachzuweisenden Moleküle (das Antigen) in der Probe binden kann, sofern vorhanden. Von dort wandert die Flüssigkeit weiter in den Kapillarbereich, wo in einem kleinen Abschnitt ein Antikörper immobilisiert wurde, der an einer anderen Stelle auf der Oberfläche des nachzuweisenden Moleküls bindet und dieses dadurch in diesem Bereich bindet und anreichert, während die Flüssigkeit weiterwandert. Durch die Anreicherung des nachzuweisenden Moleküls entsteht anhand des gebundenen Immunkonjugats je nach Immunkonjugat z. B. eine Färbung, eine Fluoreszenzfärbung oder eine magnetische Markierung.

Weitere bekannte Schnelltestverfahren sind der Flow -Through -Test (FTT), der Agglutinations-Test (AT)oder der Solid -Phase -Test (SPT). Alle diese Verfahren dienen zum schnellen Nachweis von Analyten und sind zur visuellen Auswertung im POC-Bereich geeignet.

Der Aufbau und das Design beispielhafter POC-Teststreifen wird u.a. in der WO 2011/104238 A1 oder US 2007/0231922 A1 detaillierter beschrieben. Die POC-Teststreifen können verschieden funktionelle Bereiche umfassen, wie beispielweise einen Probenbereich (engl. *application zone* oder *sample pad*), eine Markierungsregion (engl. *labeling zone*), eine Detektionsregion (engl. *detection region*) oder eine Absorptionszone (*absorbent zone*).

Nach Auftragen der Probe ist es in der Regel notwendig eine Pufferlösung oder Laufmittel auf den Teststreifen einzubringen, um die Ausbreitung der Probe über die funktionellen Bereich des Teststreifens sicherzustellen bzw. Reagenzien für den Nachweis bereitzustellen.

Im Stand der Technik ist es hierzu bekannt, in ein POC-Testystem einen flüssigkeitsgefüllten Blister zu integrieren, welcher sich stromaufwärts von dem Probenbereich befindet. Nach Auftragen der Probe wird der Blister aufgebrochen, um die Flüssigkeit (i.d.R. eine Pufferlösung) in den Teststreifen einzubringen und die Testreaktion zu unterstützen.

Ein auf einem derartigen Funktionsprinzip basierendes POC-Testsystem ist der B·R·A·H·M·S PCT direct Point of Care Test

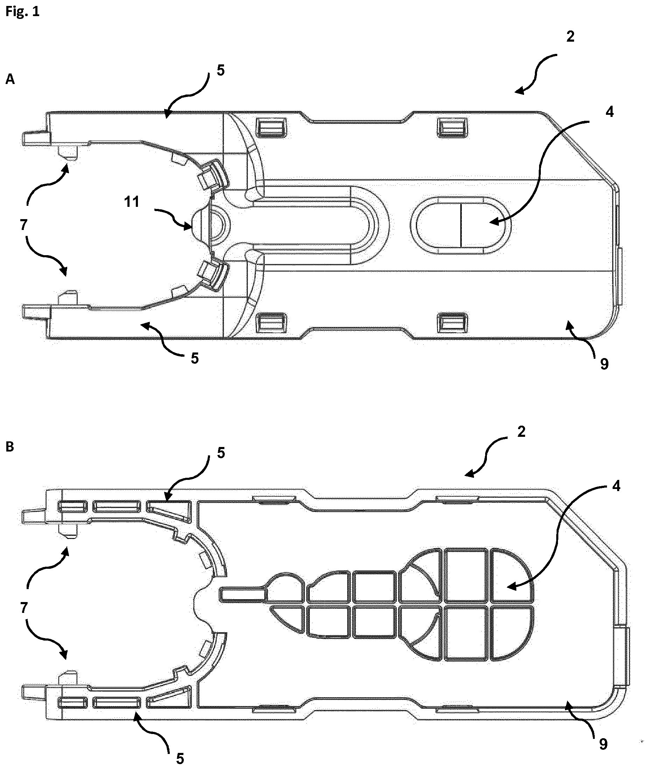

Wie in den Fig. 1-4 gezeigt, umfasst das POC-Testsytem einen Teststreifen mit funktionellen Bereichen und eine Kappe mit einem flüssigkeitsgefüllten Blister. Die Kappe ist mittels zweier Zangenarme an dem Teststreifen befestigt, sodass der Probenbereich für das Einbringen der Probe durch Kippen der Kappe zugänglich gemacht werden kann. Nach dem Auftragen der Probe wird die Kappe wieder verschlossen und die Testreaktion kann durch Entleeren des flüssigkeitsgefüllten Blisters ausgelöst werden.

Zur Bereitsstellung der POC-Testsysteme werden die Teststreifen und Kappen mit dem flüssigkeitsgefüllten Blister in der Regel separat hergestellt und anschließend zusammengesetzt. Die mit einem flüssigkeitsgefüllten Blister versehenen Kappen können an den Zangenarmen zu diesem Zweck Zapfen umfassen, welche in korrespondierenden Nuten der Teststreifen einrasten.

In bekannten Verfahren erfolgt die Zusammensetzung manuell, wobei die Komponenten, d.h. die Teststreifen und die Kappen mit den flüssigkeitsgefüllten Blistern, in einzelnen Verpackungen unsortiert bereitgestellt werden. Sowohl das Verpacken als auch die Entnahme der unsortierten Kappen führt im Fertigungsprozess zu Verzögerungen. Auch können bei nicht adäquaten Transportbedingungen die fragilen Kappen Schaden nehmen.

### Aufgabe der Erfindung

Eine Aufgabe der Erfindung war es daher eine Vorrichtung und ein Verfahren bereitzustellen, welches die Nachteile des Standes der Technik beseitigt. Insbesondere war es eine Aufgabe der Erfindung Aufbewahrungs- bzw. Transportmittel bereitzustellen, welche einen besonderen sicheren Transport sowie eine einfache, geordnete Ausgabe von Kappen für POC-Testsysteme ermöglichen.

### Zusammenfassung der Erfindung

Die Aufgabe der Erfindung wird durch die unabhängigen Ansprüche gelöst. Die abhängigen Ansprüche betreffen bevorzugte Ausführungsformen der Erfindung.

In einer bevorzugten Ausführungsform betrifft die Erfindung ein Mehrfachmagazin zum Transport, zur Aufbewahrung und/oder zur Vorhaltung einer Vielzahl von Kappen für einen POC-Teststreifen, wobei die Kappen jeweils einen Hauptkörper mit einem flüssigkeitsgefüllten Blister und zwei Zangenarme mit Zapfen zur Anbringung der Kappe an den POC-Teststreifen aufweisen und das Mehrfachmagazin an einer Innenfläche einer Vorderwand eine Reihe von zwei oder mehr sich entlang der Höhe des Mehrfachmagazins erstreckender Zungen zur Führung der Zangenarme der Kappen und auf einer gegenüberliegenden Innenfläche einer Rückwand eine Reihe von zwei oder mehr sich entlang der Höhe des Mehrfachmagazins erstreckenden Ausnehmungen zur Führung des Hauptkörpers der Kappen aufweist, sodass durch die gegenüberliegenden Zungen und Ausnehmungen eine Reihe von zwei oder mehr Fächern zur vertikalen Stapelung der Kappen gebildet werden und wobei das Mehrfachmagazin am Kopfende eine schräge Gegenlage aufweist, welche sich über die Breite des Mehrfachmagazins erstreckt und bevorzugt eine Steigung entlang der Richtung der Vorderwand zur Rückwand aufweist.

Das Mehrfachmagazin eignet sich in hervorragender Weise zum sicheren Transport und zur geordneten Bereitstellung von Kappen für einen POC-Teststreifen. Die Kappen weisen erfindungsgemäß einen Hauptkörper mit einem flüssigkeitsgefüllten Blister sowie zwei Zangenarme mit Zapfen zur Anbringung der Kappe an einem POC-Teststreifen auf, wodurch das POC-Testsystem gebildet wird. Vorliegend bezeichnet das POC-Testsystem bevorzugt die Kombination aus einem POC-Teststreifen und der Kappe mit dem flüssigkeitsgefüllten Blister. Der Begriff des POC-Teststreifens wird hingegen bevorzugt zur Kennzeichnung des Testreifen mit funktionellen Bereichen zur Bestimmung der Biomarkern verwandet. Beispielsweise kann es sich um einen immunchromatographischen Testreifen handeln, welcher zum Zweck der Detektion verschiedene funktionelle Bereiche, wie beispielweise eine Probenregion, eine Markierungsregion, eine Detektionsregion oder eine Absorptionszone umfassen kann. Ein beispielhaftes POC-Testsystem mit Teststreifen und Kappe wird in den Fig. 1-4 gezeigt.

Aufgrund der asymmetrischen Form und des fragilen Blisters stellt die Aufbewahrung und geordnete Vorhaltung der Kappen eine besondere Herausforderung dar. Bei einer Aufbewahrung in einem einfachen Behältnis oder einer Tüte können sich die Zangenarme in einer andere verhaken. Auch ist es möglich, dass der Blister durch mechanische Einflüsse bei einer ungeschützten Aufbewahrung beschädigt wird und sich zumindest teilweise vorzeitig entleert. Einerseits wird die Kappe hierdurch unbrauchbar. Falls der Fehler unerkannt bleibt, kann es zudem zu fehlerhaften Messresultaten kommen. Weiterhin erschwert eine ungeordnete Aufbewahrung eine zügige Entnahme der Kappen, um diese zusammen mit einem Teststreifen zu montieren.

Das erfindungsgemäße Mehrfachmagazin zeichnet sich durch eine optimale Verpackungsdichte bei gleichzeitig hohem Schutz der Kappen und einer besonders bequemen Handhabbarkeit aus.

So lassen sich die Kappen auf zuverlässige und einfache Weise vertikal in den dazu vorgesehenen Fächern stapeln. Die Fächer des Mehrfachmagazins werden durch Zungen und Ausnehmungen gebildet, welche an gegenüberliegenden Innenflächen des Magazins angeordnet sind. Die Zungen erstrecken sich bevorzugt über die gesamte Höhe der Innenfläche und können einen im Wesentlichen rechteckigen oder abgerundet rechteckigen Querschnitt aufweisen. Besonders bevorzugt ist die Dimensionierung der Zungen an die Form und Größe der Zangenarme der Kappen angepasst. D.h. die Zungenhöhe wird bevorzugt im Wesentlichen der Länge der Zangenarme entsprechen bzw. eine bevorzugt leicht verminderte Höhe aufweisen. Die Breite der Zunge orientiert sich bevorzugt am Abstand der Zangenarme, sodass die Zangenarme seitlich platziert geführt werden und der Hauptkörper der Kappe auf der Zunge aufsitzen kann.

Die Zungen können mithin eine optimale Führung der Fußseite der Kappen, insbesondere der dort vorliegenden Zangenarme gewährleisten. Auf der gegenüberliegenden Seite des Magazins werden die Kappen zudem durch Ausnehmungen stabilisiert, welche bevorzugt kongruent zur Form des Kopfendes der Kappe ausgebildet sind und somit analog einer Schablone das Kopfende der Kappe stabil führen. Im Sinne der Erfindung bezeichnet das Fußende der Kappe bevorzugt jenes Ende, an welchem seitliche Zangenarme zur Anbringung an dem Teststreifen vorliegen, während das Kopfende das hiervon abgewandte Ende bezeichnet, welches den Hauptkörper mit dem flüssigkeitsgefüllten Blister umfasst.

Im Falle einer Kappe mit einem rechteckigen Kopfende, kann es bevorzugt sein, dass die Vorderwand eine entsprechend rechteckige Ausnehmung bzw. Aussparung aufweist. Besonders bevorzugte Kappen, welche standardmäßig für POC-Testsyteme verwandt werden, weisen am Kopfende eine im Wesentlichen rechteckige Form auf, wobei eine Ecke abgeschrägt vorliegt. In besonders bevorzugten Ausführungsformen werden dementsprechend auch die Ausnehmungen eine im Wesentlichen rechteckigen Trapezform aufweisen.

Die beanspruchte Gestaltung des Mehrfachmagazins stellt hierbei nicht eine beliebige Designauswahl dar, welche ein Fachmann in naheliegender Weise wählen würde. Vielmehr handelt es sich um eine besondere Gestaltung und Merkmalsauswahl, welche sich von anderen denkbaren Varianten auf überraschend vorteilhafte Weise abhebt.

Als alternative Konzepte wären beispielsweise auch Trays oder Gurte denkbar, in welche die Kappen sortiert werden können. Die Erfinder haben hingegen festgestellt, dass ein Mehrfachmagazin sowohl im Hinblick auf Beladung, Entnahme, Platzbedarf, Transportsicherheit als auch Bedienerfreundlichkeit sich auf besondere Weise auszeichnet. Das Magazin ist bevorzugt derart ausgeführt, dass die Kappen eine möglichst geringe Kontaktfläche zum Magazin aufweisen, um den Abrieb zu reduzieren. Gleichzeitig wird jedoch eine hohe Transportstabilität sowie Unversehrtheit der Kappen sichergestellt.

Zudem ermöglicht der einfache und kompakte Aufbau des Mehrfachmagazins eine kostengünstige, prozesssichere Herstellung sowie eine einfache Reinigung für die Mehrwegnutzung.

Beispielsweise lassen sich bei Bereitstellungen von 14 Fächern oder mehr in welche jeweils 40 oder mehr Kappen vertikal gestapelt werden auf engstem Raum in geordneter Weise 500, 600 oder mehr Kappen beladen und sicher transportieren. Nach entsprechender Entladung kann das Magazin aufgrund des schlichten Aufbaus unter Verzicht auf fragile oder bewegliche Elemente schnell und effektiv gereinigt werden, um für eine weitere Beladung, Transport und Entladung der Kappen bereitzustehen.

In Bezug auf die Entladung zeichnet sich das erfindungsgemäße Mehrfachmagazin zudem durch eine besonders zuverlässige und prozessoptimierte Führung der Kappen aus. Erfindungsgemäß wurde erkannt, dass eine Variation der Dicke der Kappen bei vertikaler Stapelung zu einem Verdrehen bzw. Verkanten der Kappen innerhalb der Fächer führen kann. Bei standardmäßigen Kappen für POC-Teststreifen weist das Kopfende bzw. der Hauptkörper mit dem Blister zumeist eine größere Dicke auf, als das Fußende mit den Zangenarmen.

Die erfindungsgemäß vorgesehene Integration einer schrägen Gegenlage löst das Problem auf besonders zuverlässige und effektive Weise.

Die schräge Gegenlage meint bevorzugt, dass eine Auflagefläche für die Kappen am Kopfende des Magazins nicht senkrecht zur vertikalen Rück- bzw. Vorderwand verläuft, sondern sich durch einen Winkel bzw. eine Schräge auszeichnet. Die schräge Gegenlage definiert bevorzugt eine Steigung von der Innenfläche der Rückwand, an welcher sich die Zungen befinden, zur Vorderwand, an welche sich die Ausnehmungen befinden. Steigung meint hierbei bevorzugt einen Verlauf, bei welchem ein Ende der Gegenlage, welche zur Vorderwand bzw. den Ausnehmungen orientiert ist, kopfseitiger (höher) liegt als ein Ende der Gegenlage, welches zur Rückwand bzw. den Zungen orientiert ist.

Die schräge Gegenlage kann als eine vollständige Fläche das Kopfende des Mehrfachmagazins abschließen. Die schräge Gegenlage kann jedoch auch durch eine Auflagefläche mit entsprechendem Steigungswinkel gebildet werden, welche sich nur teilweise beispielsweise lediglich oberhalb der Zungen befindet.

Auch eine unterbrochene Auflagefläche ist denkbar, welche einen schrägen Steigungswinkel und mithin bei der Befüllung des Mehrfachmagazins eine gewünschte Neigung der zuerst eingebrachten Kappen sicherstellt. Beispielsweise kann die schräge Gegenlage auch durch jeweils zwei Auflageflächen für die Kappen gebildet werden, wobei eine erste Auflagefläche an der Innenfläche der Vorderwand und eine zweite Auflage an der Innenfläche der Rückwand vorliegt. Durch Anbringung der ersten Auflage in einer Position näher an der Kopfseite des Magazins, als die zweite Auflage, kann eine Steigung von der Rückwand zur Vorderwand gebildet werden. Diese führt in gewünschter Weise zu einer Neigung der ersten Lage der eingefüllten Kappen.

Für eine vertikale Stapelung der Kappen kann das Magazin beispielsweise mit dem Kopfende nach unten aufgestellt werden. Werden die Kappen nunmehr in die Fächer eingebracht, führt die schräge Gegenlage dazu, dass die ersten Kappen, welche sich im Fach unten befinden, innerhalb des Faches eine Neigung aus der Horizontalen aufweisen, welche dem Steigungswinkel der Gegenlage entspricht. Beispielweise führt ein Steigungswinkel der Gegenlage von 8° bevorzugt zu einer Schräglage bzw. Neigung von 8° der ersten Kappen gegenüber der Horizontalen. Die folgend eingeführten Kappen werden sukzessiv eine geringere Schräglage aufweisen, da für standardmäßige Kappen die Dicke des Kopfendes höher ist, als das die Dicke der seitlichen Zangenarme. Nach einer bestimmten Anzahl vertikal gestapelten Kappen liegt keine Neigung bzw. Schräglage mehr vor, sodass die letzten Lagen der Kappen eine im Wesentlichen horizontale Ausrichtung erfahren.

Zur Entladung der Kappen kann das Mehrfachmagazin bevorzugt mit dessen Fußseite nach unten orientiert werden, sodass die Kappen mittels der Schwerkraft oder durch zusätzlich aktive Beförderung entnommen werden können.

Mittels der schrägen Gegenlage kann hierbei gewährleistet werden, dass die letzte Lage der eingefüllten Kappen - mithin die erste Lage der Kappen, welche Entladen wird - optimal horizontal positioniert vorliegt. Entsprechend ihrer horizontalen Ausrichtung fallen die Kappen bevorzugt mit dem Kopf- und Fußende gleichzeitig auf eine Unterlage.

Versuche zeigen, dass mittels der schrägen Gegenlage nicht nur eine erleichterte und zügigere Entnahme möglich ist, sondern zudem Abrieb an den Kappen aufgrund von Schrägstellungen im Bereich der Entnahme vermieden werden können.

In einer bevorzugten Ausführungsform weist die schräge Gegenlage einen Steigungswinkel von 5° - 15°, bevorzugt von 6° - 10° auf, besonders bevorzugt von ungefähr 8°. Der Steigungswinkel wird bevorzugt in Bezug auf die horizontale Ebene angegeben, welche senkrecht auf den vertikalen Rück- bzw. Vorderwänden steht. Auch Zwischenwerte aus den vorgenannten Bereichen können bevorzugt sein, wie beispielsweise 6°, 7°, 8°, 9°, 10°, 11°, 12°, 13°, 14° oder auch 15° sowie Kombination der Zwischenwerte untereinander oder mit den bevorzugten Bereichsgrenzen. Die vorgenannten Winkel sind besonders geeignet, um eine vertikale Stapelung der Kappen zu ermöglichen, welche bei optimaler Platzausnutzung eine zügige und sichere Entladung gewährleistet.

In einer bevorzugten Ausführungsform der Erfindung ist die Zungenhöhe zwischen 0,5 mm und 3 mm höher, als die Länge der Zangenarme der Kappe, sodass bei einer Stapelung der Kappen im Mehrfachmagazin die Enden der Zangenarme das Mehrfachmagazin nicht berühren. Die Länge der Zangenarme wird bevorzugt von der Ebene des niedrigsten (d.h. zur Fußseite gewandten) Punktes des Hauptkörpers der Kappe bestimmt und entspricht dem Abstand bis zum äußersten Ende der Zangenarme. Die Zungenhöhe hingegen entspricht der Distanz von dem höchsten Punkt der Zunge (welcher in den Magazininnenraum ragt) bis zur Innenfläche der Rückwand. Bei einer Zungenhöhe, welche gleich der Länge der Zangenarme ist, werden die äußersten Enden der Zangenarme die Seiteninnenwand berühren, insofern das Magazin auf die Rückwand gelegt wird.

In der bevorzugten Ausführungsform wird eine derartige Berührung dadurch vermieden, dass die Zungenhöhe geringfügig, d.h. bevorzugt mit einer Toleranzspanne von 0,5 mm bis 3 mm höher ist als die Länge der Zangenarme. Auch Zwischenwerte aus den vorgenannten Bereichen können bevorzugt seien, wie beispielsweise 0,6 mm, 0,7 mm, 0,8 mm, 0,9 mm, 1,0 mm, 1,2 mm, 1,4 mm, 1,6 mm, 1,8 mm, 2,0 mm, 2,2 mm, 2,4 mm, 2,6 mm oder 2,8 mm sowie Kombination der Zwischenwerte untereinander oder mit den bevorzugten Bereichsgrenzen. Falls der Hauptkörper der Kappe im mittleren Bereich beispielsweise einen Ausstülpung aufweist, wird die Kappe lediglich auf der Ausstülpung aufsetzen, ohne dass eine Berührung mit der Rückwand erfolgt.

Die Erfinder konnten feststellen, dass die vorgenannten Parameterbereiche besonders gute Ergebnisse in Bezug auf eine schnelle Be- und Entladung ohne Verkanten erzielen und gleichzeitig beim Transport Beschädigungen und Abrieb wirksam vermeiden.

Standardisierte Kappen für POC-Teststreifen, beispielsweise Kappen für den B·R·A·H·M·S PCT direct Point of Care Test weisen eine bevorzugte Länge der Zangenarme von ca. 10 -20 mm auf, bevorzugt ca. 13-18 mm.

In einer bevorzugten Ausführungsform beträgt die Zungenhöhe daher zwischen 10 mm und 25 mm, bevorzugt zwischen 14 mm und 22 m. Mittels dieser Werte können die vorgenannten Toleranzspannen für das Aufliegen der Spitzen der Zangenarme und ein sicheres Führen der Kappen sichergestellt werden.

In einer weiteren bevorzugten Ausführungsform der Erfindung ist die Zungenbreite zwischen 1 mm und 2 mm, bevorzugt zwischen 1,5 mm und 2 mm, schmaler, als der Abstand der Zapfen der Zangenarme der Kappen, sodass bei einer Stapelung der Kappen im Mehrfachmagazin die Zapfen der Zangenarme beidseitig mit einer Toleranz von 0,5 mm bis 1 mm, bevorzugt von 0,75 bis 1 mm, geführt werden.

Auch Zwischenwerte aus den vorgenannten Bereichen können bevorzugt sein. Beispielsweise kann der Abstand der Zapfen der Zangenarme die Zungenbreite um 1,1 mm, 1,2 mm, 1,3 mm, 1,4 mm, 1,7 mm, 1,8 mm oder 1,9 überragen. Ganz besonders bevorzugt ist eine Zungenbreite, welche um ca. 1,7 mm bis 1,8 mm kleiner ist, als der Abstand der Zapfen der Zangenarme, sodass die Zapfen der Zangenarme beidseitig mit einer Toleranz ca. 0,85 - 0,9 mm geführt werden.

Begriffe wie im Wesentlichen, ungefähr, etwa, ca. etc. beschreiben bevorzugt einen Toleranzbereich von weniger als ± 20%, bevorzugt weniger als ± 10 %, noch stärker bevorzugt weniger als ± 5% und insbesondere weniger als ± 1 %. Angaben von im Wesentlichen, ungefähr, etwa, ca. etc. offenbaren und umfassen stets auch den exakten genannten Wert.

Die vorgenannten Toleranzspannen führen zu optimalen Bedingungen, sowohl in Bezug auf ein Beladen, einen Transport als auch ein Entladen des Mehrfachmagazins.

Insbesondere ist die Handhabung bei manueller Beladung fehlerfrei und unkompliziert. Die seitliche Toleranz zur Führung der Zangenarme erlaubt zudem ein hinreichendes Spiel beim Befüllen, sodass hohe Befüll-Geschwindigkeiten erreicht werden können. Gleichzeitig wird ein Verkanten oder Kippeln der Kappen vermieden und eine gleichmäßige Kappenausrichtung erzielt.

In Bezug auf den Transport zeigen Versuche, dass bei vollständiger Befüllung und einem über einen längeren Zeitraum erfolgten Rütteln bzw. Schütteln des Magazins, die vorgenannten Parameter eine besonders sichere Lagerung gewährleisten. So ist die Kappen-Fixierung ausgezeichnet und weder Beschädigungen des Blisters, noch ein Abrieb sind feststellbar.

Weiterhin zeichnet sich die Ausführungsform in besonderem Maße für die eine praktikable und zügige Entladung aus. Zum einen ist nahezu keine Blockieren bzw. Verkanten der Kappen feststellbar. Weiterhin ist vorteilhafterweise eine passive Entladung aufgrund der Schwerkraft möglich, wobei jedoch eine zumindest leichte aktive Unterstützung mit Kräften im Bereich von ca. 1 Newton zu einem besonders schnellen Abspenden führen.

Die Zungenbreite wird entsprechend der Ausführungsform bevorzugt an die Dimensionierung der Kappen angepasst. Standardisierte Kappen für POC-Teststreifen, beispielsweise die Kappen für den B·R·A·H·M·S PCT direct Point of Care Test, weisen einen bevorzugten Abstand der Zapfen der Zangenarme von ca. 8 mm bis 15 mm, bevorzugt ca. 11,5 mm, auf.

In einer bevorzugten Ausführungsform der Erfindung beträgt die Zungenbreite daher zwischen 8 mm und 12 mm, bevorzugt zwischen 9 mm und 10,5 mm.

In einer bevorzugten Ausführungsform weist das Mehrfachmagazin eine Reihe von 5 - 30, bevorzugt 8 - 20 Fächer, ganz besonders bevorzugt 12 - 16 Fächer, insbesondere 14 Fächer zur vertikalen Stapelung der Kappen auf.

In einer bevorzugten Ausführungsform ist das Mehrfachmagazin in der Höhe derart dimensioniert, dass in jedem Fach zwischen 35 und 50, bevorzugt zwischen 40 und 45 Kappen vertikal gestapelt werden können.

Mehrfachmagazin meint bevorzugt ein Behältnis zur Aufbewahrung, zum Transport und/oder Vorhaltung der Kappen in mindestens zwei oder mehr Fächern. Bevorzugt ist das Mehrfachmagazin im Wesentlichen quaderförmig, wobei eine abgeflachte Quaderform besonders bevorzugt ist.

Das Mehrfachmagazin umfasst bevorzugt eine Vorderwand bzw. Rückwand, welche die Höhe und Breite aufspannen, sowie zwei schmale Seitenwände (Höhe mal Tiefe) und ein Kopf- bzw. Fußende. Das Fußende stellt bevorzugt eine ggf. verschließbare Öffnung zum Be- und Entladen der Kappen dar, während am Kopfende eine schräge Gegenlage vorliegt.

Im Sinne der Erfindung entspricht die Höhe des Mehrfachmagazins bevorzugt jener Ausrichtung entlang welcher die Kappen vertikal gestapelt werden. Bevorzugt wird die Höhe von den Außenmaßen des Magazins bestimmt, sodass die Höhe der Fächer um die Wandstärke, also beispielsweise um 0,5 cm bis 2 cm geringer ist.

Standardisierte Kappen für POC-Testsytsmen, wie beispielsweise der B·R·AH·M·.S PCT direct Point of Care Test, weisen an ihrem größten Querschnitt eine Dicke von ca. 5-7 mm auf. Für eine vertikale Stapelung von 35 bis 50 Kappen, bevorzugt von 40 bis 45 Kappen ist daher eine Höhe des Magazins von ca. 20 cm bis 40 cm, bevorzugt von ca. 25 bis 30 cm besonders bevorzugt.

Im Sinne der Erfindung entspricht die Breite des Mehrfachmagazins bevorzugt der Dimensionierung des Magazins entlang welcher sich die Reihe von zwei oder mehr vertikalen Fächer erstreckt. Die gesamte Breite des Magazins wird mithin bevorzugt durch die Anzahl und Breite der vertikalen Fächer bestimmt.

Die Breite der Fächer orientiert sich bevorzugt an der Breite der Kappen. Standardisierte Kappen für POC-Teststreifen, beispielsweise Kappen für einen B·R·A·H·M·S PCT direct Point of Care Test weisen eine bevorzugte Breite von ca. 15 mm bis 25 mm, besonders bevorzugt ca. 20 mm auf.

Die Fächer werden wie hierin beschrieben durch gegenüberliegen Zungen und Ausnehmungen in den Innenflächen der Vorder- bzw. Rückwände gebildet. Die periodische Beabstandung der Ausnehmungen bzw. Zungen entlang der Breite der Innenflächen gibt mithin die Breite der vertikalen Fächer vor. Besonders bevorzugte Werte für die Breite der vertikalen Fächer liegen zwischen 15 und 30 mm, besonders bevorzugt zwischen 20 und 25 mm, ganz besonders bevorzugt bei ca. 23 mm.

Die periodischen Abstände zwischen den Ausnehmungen bzw. Zungen und somit die Breite der Fächer ist ebenfalls, um eine geringfügige Toleranzspanne von ca. 2-5 mm, bevorzugt ca. 3 mm, größer als die Breite der Kappen. Hierdurch wird eine optimale Packungsdichte, bei gleichzeitig sicherem Transport und komplikationsfreier Be- und Entladung erzielt.

Die Breite des Mehrfachmagazins wird wiederum bevorzugt durch das Außenmaß bestimmt sodass die gesamte Breite aller Fächer der Reihe, um die Stärke der äußeren Wände, also beispielsweise um 0,5 cm bis 2 cm geringer ist. Besonders bevorzugte Breiten des Mehrfachmagazins betragen zwischen ca. 15 cm und 50 cm, ganz besonders bevorzugt ca. 25 cm bis 40 cm, am meisten bevorzugt von ca. 33,5 cm.

Im Sinne der Erfindung bezeichnet die Tiefe des Mehrfachmagazins bevorzugt die Dimensionierung des Magazins orthogonal zur Breite und Höhe. Die Tiefe des Magazins bzw. die Tiefe der vertikalen Fächer ist bevorzugt an die Gesamtlänge der Kappen angepasst.

Standardisierte Kappen für POC-Teststreifen, wie beispielsweise der B·R·AH·M·S PCT direct Point of Care Test, weisen eine bevorzugte Gesamtlänge von ca. 40 mm bis 70 mm, besonders bevorzugt von 50 -60 mm, ganz besonders bevorzugt von ca. 55 mm auf.

Die Tiefe der vertikalen Fächer bestimmt sich bevorzugt durch den größten Abstand zwischen den Innenflächen der Vorder- bzw. Rückwand des Magazins, an welchen die Ausnehmungen bzw. Zungen angeordnet vorliegen. Bevorzugte Werte für die Tiefe der vertikalen Fächer betragen beispielsweise ca. 45 mm bis 70 mm, wobei es bevorzugt ist die Tiefe der Fächer so zu wählen, dass eine Toleranz für die Kappen, beispielsweise von ca. 1-3 mm, verbleibt.

Die Gesamttiefe des Magazins bestimmt sich bevorzugt als Außenmaß, sodass die Tiefe des Magazins um die Stärke der Magazinwände, also beispielsweise einen Faktor von 0,5 cm bis 2 cm höher ist, als die Tiefe der Fächer. Bevorzugte Tiefen des Magazins liegen in einem Bereich von ca. 45 bis 75 mm, besonders bevorzugt im Bereich von ca. 60 cm bis 65 cm.

In einer besonders bevorzugten Ausführungsform weist das Magazin eine Höhe von 20 cm bis 40 cm, bevorzugt 25 bis 30 cm, eine Breite von 15 cm bis 50 cm, bevorzugt von 25 cm bis 40 cm und eine Tiefe von 45 bis 75 mm, bevorzugt 60 bis 65 cm, auf. Hierdurch lassen sich beispielsweise 12-16 Fächer in einer Reihe realisieren in welche 35- 50 Kappen vertikal gestapelt werden können.

Ein derart dimensioniertes Magazin erlaubt mithin den Transport und die Vorhaltung von 400, 500, 600 oder mehr Kappen auf engstem Raum. Zudem entsprechen die vorgenannten Maße der Breite und Höhe bevorzugt ca. einem Viertel einer Europalette (1200 mm x 800 mm), sodass ein platzeffektiver Transport einer Vielzahl von Mehrfachmagazinen auf genormten Palletten möglich ist.

Beispielsweise können mehrere Magazine in einer Stapelkiste transportiert werden, wobei es bevorzugt sein kann, die Magazine zusätzlich in Verpackungsmaterial, beispielsweise foliert und stoßgeschützt, in die Stapelkiste einzubringen.

Bevorzugte Maße der Stapelkiste können durch das Standard-Euro-Palettenmaß für den Transport vom Kappenhersteller zum Verarbeitungsort von 1,20 x 0,80 m bzw. einem geraden Teile davon, beispielsweise 0,60 x 0,40 m vorgeben werden. Mit den bevorzugten genannten Außenmaßen für die Magazin können, auch bei Verwendung von adäquatem Verpackungsmaterial, beispielsweise zwei Magazine nebeneinander in ein bis x Lagen übereinander je Stapelkiste transportiert werden können.

Vorteilhafterweise wird insbesondere bei Verwendung von Kunststoff als Material für die Magazine auch für derart befüllte Stapelkisten ein Gesamtgewicht gewährleistet, welches normativen Anforderungen an zu hebende Massen auch für weibliche Arbeitskräfte genügt. Bei den besonders bevorzugten Abmaßen für die Magazin und beispielsweise eine Befüllung von ca. 600 Kappen je Magazin sowie fünf Lagen mit je zwei Magazinen nebeneinander kann pro Stapelkiste ein Bruttogewicht der Kiste von etwa weniger als 30 kg sichergestellt werden. Ein Transport der Stapelkisten lässt sich mithin von zwei Arbeitskräften gemäß Lastenhandhabungsverordnung bewerkstelligen.

In einer weiteren bevorzugten Ausführungsform weist das Mehrfachmagazin zusätzliche Trennwände auf, welche bevorzugt jeweils einen Abschnitt von ein, zwei, drei oder mehr Fächern voneinander trennen. Die Trennwände erstrecken sich bevorzugt entlang der gesamten Höhe des Magazins von einer Innenfläche der Rückwand zur gegenüberliegenden Innenfläche der Vorderwand. Während die Zungen und Ausnehmungen bereits eine überaus stabile Lagerung der Kappen bewirken, führen die Trennwände zu einer weiteren Stabilisierung des Mehrfachmagazins. Auch können die Trennwände beispielsweise Kompartimente von zwei, drei oder mehr nebeneinanderliegenden Fächern definieren, welche zeitgleich be- oder entladen werden können.

In einer bevorzugten Ausführungsform weist das Mehrfachmagazin einen, bevorzugt zwei Schieber zum wahlweisen Öffnen bzw. Verschließen des Fußendes auf. Besonders bevorzugt ist die Breite des einen oder der zwei oder mehr Schieber geringfügig schmaler, als die Tiefe des Mehrfachmagazins. Die Breite des Schiebers beträgt beispielsweise zwischen 35 mm und 60 mm, wobei die Gesamtlänge der Schieber bevorzugt der Breite des Mehrfachmagazins entspricht. Der oder die Schieber können auch mehrere Streben bilden, sodass ein teilweiser Verschluss des Fußendes erfolgt.

Zur Gewährleistung der Ausrichtung der Schieber können Führungen vorliegen. Beispielsweise können diese mittels der Trennwände gebildet werden, welche entsprechende Durchgänge aufweisen.

Im Falle von zwei Schiebern ist es bevorzugt, dass deren Länge ca. einer halben Breite des Mehrfachmagazins entspricht, sodass die Schieber symmetrisch die Fußseite des Mehrfachmagazins öffnen und verschließen können.

Die Schieber dienen bevorzugt dem Verschluss des Mehrfachmagazins nachdem die Kappen eingebracht wurden und gewährleisten einen sicheren Transport.

Im Gegensatz zu alternativen Verschlüssen, wie beispielsweise einer Folie, zeichnen sich die Schieber insbesondere durch ihre mehrfache Verwendbarkeit aus. Auch ist das Öffnen bzw. Schließen mittels zweier Schieber besonders leicht und kann zudem in einen automatisierten Prozess integriert werden.

Für die Fertigung des Mehrfachmagazins können unterschiedliche Materialien verwendet werden. Beispielsweise eignen sich Metalle, u.a. Aluminium, Eisen, Kupfer, und Metalllegierungen, u.a. Bronze, Messing, Stahl oder Edelstahl. Diese Materialien weisen eine hohe Beständigkeit und eine ansprechende Optik auf, wobei Aluminium aufgrund seiner Leichtigkeit besonders geeignet ist.

In einer besonders bevorzugten Ausführungsform ist das Mehrfachmagazin aus Kunststoff gefertigt, besonders bevorzugt aus Acrylnitril-Butadien-Styrol (ABS) oder Polyethylen (PE), ganz besonders bevorzugt aus PE-12.

Kunststoffe bezeichnen im üblichen Sinne Werkstoffe, welche aus Polymeren aufgebaut sind und sich durch eine hohe Formbarkeit auszeichnet. Bei dem Kunststoff kann es sich beispielsweise um Polyethylen (PE), Polypropylen (PP), Polyvinylchlorid (PVC), Polystyrol (PS), Polyurethan (PU/PUR) oder Polyethylenterephthalat (PET) handeln. Besonders bevorzugt ist als Kunststoff Acrylnitril-Butadien-Styrol (ABS) oder Polyethylen (PE).

Kunststoff zeichnet sich durch ein geringes Materialgewicht aus, wodurch der Transport erleichtert wird. Zudem haben sich die Kunststoffe beispielsweise gegenüber Metall als besonders schonend für eine sichere Lagerung der Kappen erwiesen. So verringert sich unter Verwendung der Kunststoffe ein Abrieb. Auch das Risiko der Beschädigungen fragiler Bestandteile der Kappe ist vermindert.

In einem weiteren Aspekt betrifft die Erfindung eine Verwendung eines Mehrfachmagazins gemäß der Erfindung oder einer bevorzugten Ausführungsform hiervon zur Aufbewahrung, zum Transport und/oder Vorhaltung einer Vielzahl von Kappen für einen POC-Teststreifen.

In einem weiteren Aspekt betrifft die Erfindung zudem einen Kit umfassend ein Mehrfachmagazin gemäß der Erfindung oder einer bevorzugten Ausführungsform hiervon sowie eine Vielzahl von Kappen für einen POC-Teststreifen, wobei die Kappen jeweils einen Hauptkörper mit einem flüssigkeitsgefüllten Blister und zwei Zangenarme zur Anbringung der Kappe an dem POC-Teststreifen aufweisen und wobei die Vielzahl von Kappen bevorzugt in den Fächern des Mehrfachmagazins vertikal gestapelt vorliegen.

Der Fachmann erkennt, dass bevorzugte Ausführungsformen und Vorteile, welche im Zusammenhang mit dem Mehrfachmagazin zum Transport und/oder Vorhaltung einer Vielzahl von Kappen für einen POC-Teststreifen offenbart wurden sich gleichermaßen auf die beanspruchte Verwendung bzw. den Kit übertragen. Insbesondere gelten die bevorzugten Ausführungsformen für das Mehrfachmagazin, wie für die Kappen gleichermaßen für den Kit bzw. die Verwendung.

Die Verwendung betrifft eine Aufbewahrung, zum Transport und/oder Vorhaltung einer Vielzahl von Kappen für einen POC-Teststreifen. Das Kit umfasst neben dem Mehrfachmagazin eine Vielzahl von Kappen für einen POC-Teststreifen.

Die Kappen sind wie vorgehend beschrieben insbesondere durch einen Hauptkörper gekennzeichnet, welcher einen flüssigkeitsgefüllten Blister umfasst. Blister meint im Sinne der Erfindung bevorzugt ein flüssigkeitsdichtes Behältnis, welches durch mechanisches Einwirken gezielt beschädigt werden kann, sodass ein kontrollierter Austritt der Flüssigkeit erfolgt. Zu diesem Zweck kann der Hauptkörper oberhalb des Blisters auch eine Aussparung aufweisen, sodass der Blister mittels einer gezielten Fremdeinwirkung geöffnet werden kann. Ein Blister kann beispielsweise ein Beutel aus einer Plastik- oder Aluminiumverbundfolie sein. Bevorzugt umfasst der Blister als Flüssigkeit eine Pufferlösung, welche zur Durchführung des Testverfahrens auf den POC-Teststreifen aufzubringen ist. Bei der Pufferlösung kann es sich beispielsweise um ein Laufmittel für einen *lateral flow test* handeln. Das Fassungsvermögen des Blisters liegt bevorzugt im Mikroliterbereich, also bevorzugt zwischen 1 µl und 1000 µl, bevorzugt zwischen 10 µl und 500 µm, besonders bevorzugt zwischen 50 µl und 300 µl.

Die Kappen sind für das Aufsetzen auf einen POC-Teststreifen konfiguriert und umfassen zu diesem Zweck seitliche Zangenarme mit Zapfen, welche in korrespondierende Aussparungen eines POC-Teststreifens greifen können.

In bevorzugten Ausführungsformen ist die Form und Geometrie der Kappen ebenfalls wie vorgehend beschrieben. Beispielsweise können die Kappen eine bevorzugte Länge der Zangenarme von 10-20 mm, einen Abstand der Zapfen der Zangenarme von 8-15 mm sowie eine Gesamtbreite von ca. 15-25 mm und Gesamtlänge von ca. 40-70 mm aufweisen. Bevorzugt sind die Kappen aus Kunststoff gefertigt, wobei der Hauptkörper eine im Wesentlichen rechteckige Form mit einer Querschnittsdicke von 4-8 mm, bevorzugt 5-7 mm aufweist.

Ganz besonders bevorzugt handelt es sich um die in den Fig. 1 bis 4 gezeigten Kappen, welche für einen der B·R·A·H·M·S PCT direct Point of Care Testkonfiguriert sind und kommerziell von Thermo Fisher Scientific vertrieben werden.

In einer bevorzugten Ausführungsform des Kits liegt die Vielzahl von Kappen in den Fächern des Mehrfachmagazins vertikal gestapelt vor.

In einer weiteren bevorzugten Ausführungsform des Kits liegen 35-50 Kappen, besonders bevorzugt 40-45 Kappen in den Fächern des Mehrfachmagazins vertikal gestapelt vor.

In einem weiteren Aspekt betrifft die Erfindung ein Verfahren zur Montage einer Vielzahl von POC-Testsystemen umfassend die Schritte
a. Bereitstellung eines erfindungsgemäßen Mehrfachmagazins oder bevorzugten Ausführungsformen davon, wobei innerhalb des Mehrfachmagazins eine Vielzahl von Kappen für einen POC-Teststreifen mit einem Hauptkörper mit einem flüssigkeitsgefüllten Blister und zweier Zangenarme mit Zapfen zur Anbringung an einen POC-Teststreifen,
b. Bereitstellung einer Vielzahl von POC-Teststreifen mit beidseitigen Nuten zur Aufnahme der Zapfen der Kappen
c. Individuelle Entladung der Kappen aus dem Mehrfachmagazin auf einen Montagetisch und/oder ein Transportband
d. Aufsetzen der Kappe auf den POC-Teststreifen, wobei die Zapfen der Zangenarme in die beidseitigen Nuten des Teststreifens einrasten.

Der Fachmann erkennt, dass bevorzugte Ausführungsformen und Vorteile, welche im Zusammenhang mit dem Mehrfachmagazin sowie dem erfindungsgemäßen Kit offenbart wurden sich gleichermaßen auf das Verfahren übertragen.

Insbesondere ist es bevorzugt, die offenbarten bevorzugten Ausführungsformen des Mehrfachmagazins bzw. der Kappen bereitzustellen bzw. zu verwenden.

Die vorteilhafte Verwendung des Mehrfachmagazins zeigt sich insbesondere in der individuellen Entladung der Kappen aus dem Mehrfachmagazin auf einen Montagetisch und/oder ein Transportband. In manueller Hinsicht kann dies besonders schnell und zuverlässig erfolgen. Zudem erlaubt die Verwendung eines erfindungsgemäßen Mehrfachmagazins eine Automatisierung des Prozesses und somit deutlich Erhöhung der Stückzahl.

Zum einen liegen die Kappen bereits sortiert und in gleichmäßiger Ausrichtung in den vertikalen Fächern vor. Dies erleichtert und beschleunigt das weitergehende Prozessieren. Zum anderen kann die individuelle Entnahme vorteilhafterweise entweder ausschließlich auf Basis der Schwerkraft oder aber mit leichter aktiver Unterstützung, beispielsweise eines Kolbens, erfolgen.

Die Zungen und Ausnehmungen stellen eine Führung der Kappen innerhalb des Faches sicher, sodass ein Verkippen bzw. Verkanten vermieden wird. Zudem gewährleistet die schräge Gegenlage eine optimale horizontale Ausrichtung der Kappen am Fußende, welche den Weitertransport, beispielsweise mittels eines automatischen Schiebers auf effiziente Weise ermöglicht.

In einer bevorzugten Ausführungsform erfolgt die individuelle Entladung der Kappen aus dem Mehrfachmagazin ausschließlich aufgrund der Schwerkraft. Zu diesem Zweck wird das Mehrfachmagazin bevorzugt mit der Fußseite nach unten aufgestellt. Ein Schieber beispielsweise mit einer Aussparung für die Kappen kann eine gezielte Mitnahme der Kappen steuern.

Die seitlichen Schieber können bevorzugt den jeweiligen Verschluss der Fächer steuern, sodass lediglich ein oder ggf. mehrere bestimmte Fächer für die individuelle Entladung der Kappen fußseitig geöffnet sind.

In einer bevorzugten Ausführungsform des Verfahrens wird die individuelle Entladung der Kappen aus dem Mehrfachmagazin durch Verwendung eines Kolben unterstützt, welcher die in einem Fach des Mehrfachmagazins vertikal gestapelten Kappen ausstößt. Hierbei ist es bevorzugt, dass das Mehrfachmagazin entlang der Gravitation mit der Fußseite nach unten orientiert ist. Vorteilhafterweise reichen bei einer derartigen Orientierung bereits eine geringfügige Unterstützung mit Kräften von 1 Newton (100g) oder weniger um die individuelle Entladung deutlich zu beschleunigen.

Es kann aber auch bevorzugt sein, dass das Mehrfachmagazins nicht mit der Fußseite nach unten orientiert ist. Die Verwendung eines Kolbens zum Ausstoß der Kappen führt in dieser Hinsicht zu einer erhöhten Flexibilität.

In einer bevorzugten Ausführungsform des Verfahrens kann das Mehrfachmagazin daher beispielsweise auch stehend von unten mittels eines bzw. mehrerer Schieber entladen werden. Hierzu kann es bevorzugt sein die Kappe/n jeweils um eine Kappenbreite nach oben zu schieben, und mittels einer automatisierten Entnahmevorrichtung, beispielsweise einer Pick & Place-Einheit, zu entnehmen und weiter zu prozessieren. Der Entladevorgang des Mehrfachmagazins lässt sich mithin flexible an konstruktive Anforderung bei der Einbettung in einen automatisierten Prozess anpassen.

In einer bevorzugten Ausführungsform des Verfahrens erfolgt die Entladung der Kappen dadurch, dass das Mehrfachmagazin mit der Fußseite auf einen Abstandshalter gestellt wird und eine individuelle Entnahme der Kappen mittels eines Schiebers erfolgt, welcher eine Aussparung zur Aufnahme der Kappen aufweist.

Die Aussparung kann beispielsweise eine rechteckige Form aufweisen, deren Länge bzw. Breite der Gesamtlänge bzw. Gesamtbreite der Kappen entspricht. Unterhalb der Aussparung liegt bevorzugt zumindest teilweise eine Auflagefläche vor, beispielsweise in Form einer rechteckigen inneren Umrandung. Bevorzugt ist der Schieber mit der Aussparung derart dimensioniert, dass bei Führung des Schiebers unter ein Fach eine Kappe mittels der Schwerkraft oder ggf. zusätzlich unterstützt unter Verwendung eines Kolbens vom Schieber mitgenommen wird.

Die Verwendung eines Schiebers erlaubt eine besonders effiziente manuelle Entnahme und kann zudem vorteilhafterweise für eine Automatisierung des Prozesses eingesetzt werden. Hierzu kann beispielsweise eine computergesteuerte Kontrolle des Schiebers vorgesehen sein, welche den Schieber periodisch unter eines der Fächer des Mehrfachmagazins fährt, um eine zügige, Einzelentladung zu bewirken. Nach vollständiger Entladung der Kappen eines Faches wird der Schieber zu einem benachbarten Fach verschoben, um dieses zu entladen.

Bei individueller Entladung beispielsweise auf ein Transportband erfolgt ein besonders effektiver Prozess einer Bereitstellung der Kappen für die anschließende Montage der Kappen auf den POC-Teststreifen.

In bevorzugten Ausführungsformen betrifft die Erfindung auch ein System aus einer Steuereinheit und dem Mehrfachmagazin, wobei die Steuereinheit eine Datenverarbeitungseinheit sowie Aktuatoren zur automatisierten Steuerung des einen oder mehr Schieber zum Öffnung und Schließen des Mehrfachmagazins, des einen oder mehr Schieber mit einer Aussparung zur Mitnahme der Kappen und/oder des einen oder mehr Kolben zum Ent- oder Beladen des Magazins umfasst.

Die Datenverarbeitungseinheit ist bevorzugt eine Einheit, welche zum Empfang, Senden, Speichern und/oder Verarbeiten von Daten, geeignet und konfiguriert ist. Die Datenverarbeitungseinheit umfasst bevorzugt einen integrierter Schaltkreis, ein Prozessor, ein Prozessorchip, ein Mikroprozessor und/oder einen Mikrokontroller zur Verarbeitung von Daten, sowie einen Datenspeicher, beispielsweise eine Festplatte, einen *random access memory* (RAM), einen *read-only memory* (ROM) oder auch einen *flash memory* zur Speicherung der Daten. Besonders bevorzugt ist die Datenverarbeitungseinheit ein Personal Computer (PC), ein Laptop, ein Tablet oder dergleichen, welche neben Mitteln zum Empfang, Senden, Speichern und/oder Verarbeiten von Daten auch ein Anzeigen der Daten sowie Eingabemittel umfassten, wie beispielsweise ein Keyboard, ein Maus, ein Touchscreen etc.

Die Datenverarbeitungseinheit wird bevorzugt dafür genutzt, um einen automatisierten Prozess beispielsweise zum Ent- oder Beladen der Kappen in das Mehrfachmagazin zu steuern. Zu diesem Zweck kann auf der Datenverarbeitungseinheit bevorzugt eine Software, Firmware bzw. ein Computerprogramm gespeichert vorliegen, welches Befehle umfasst, die Prozessschritte, ggf. unter Vorgabe von Prozessparametern, ausführen.

Die Montage der Kappen auf den POC-Teststreifen kann automatisiert oder manuell erfolgen. Vorzugsweise beinhaltet sie ein Einrasten der Zapfen der Zangenarme in entsprechende Nuten des POC-Teststreifens.

POC-Teststreifen meint bevorzugt Durchfluss-Testreifen zur Bestimmung von Biomarkern und umfasst insbesondere immunchromatographische Teststreifen, welche beispielsweise über eine Antikörperbindung ein lösliches Biomolekül z.B. ein Hormon oder ein Protein in Blut, Urin oder Speichel immobilisieren und nachweisen.

Die POC-Teststreifen können verschieden funktionelle Bereiche umfassen, wie beispielweise einen Probenbereich (engl. *application zone* oder *sample pad), ei*ne Markierungsregion (engl. *labeling zone*), eine Detektionsregion (engl. *detection region*) oder eine Absorptionszone (*absorbent zone*). Die Funktionsweise und mögliche Designs von POC-Teststreifen sind dem Fachmann bekannt. Beispielhaft sei auf die WO 2011/104238 A1 oder US 2007/0231922 A1 verwiesen.

Die funktionellen Bereiche des POC-Teststreifen sind bevorzugt in einem Gehäuse eingefasst, welche bevorzugt eine im Wesentlichen abgeflachte Streifen- bzw. Quaderform aufweist. Beispielsweise kann das Gehäuse der POC-Teststreifen eine Länge von 4 cm bis 12 cm, vorzugsweise 6 cm bis 10 cm, eine Breite von 1 cm bis 3 cm, vorzugsweise von 1,5 cm bis 2,5 cm und eine Dicke von 0,1 cm bis 1 cm, vorzugsweise 0,2 bis 0,7 cm, aufweisen. Die Nuten zur Anbringen der Kappen sind vorzugsweise seitlich im vorderen Bereich des Gehäuses des POC-Teststreifens angeordnet.

Ein besonders bevorzugter POC-Teststreifen ist der B·R·A·H·M·S PCT direct Point of Care Test, welcher beispielsweise kommerziell von der B.R.A.H.M.S. GmbH (ThermoFisher Scientific) vertrieben und in den Fig. 1 bis 4 illustriert ist.

### FIGUREN

Im Folgenden soll die Erfindung an Hand von Figuren näher erläutert werden, ohne auf diese beschränkt zu sein.

### Kurzbeschreibunq der Abbildungen

- Fig. 1: Schematische Draufsicht (A) und Schnittdarstellung (B) einer bevorzugten Ausführungsform einer Kappe
- Fig. 2: Schematische Darstellung einer bevorzugten Ausführungsform eines POC-Testsystems aus Kappe und POC-Teststreifen im geöffneten Zustand (A) und geschlossen Zustand (B)
- Fig. 3: Schematische Darstellung einer bevorzugten Ausführungsform eines Mehrfachmagazins in einer 3D Vorderansicht (A) und einer Schnittansicht (B)
- Fig. 4: Schematische Detailansicht der Fixierung einer Kappe innerhalb der Fächer des Mehrfachmagazins
- Fig. 5: Schematische Schnittdarstellung eines mit Kappen gefüllten Magazins zur Illustration der Lagerung der Kappe mit einer schrägen Gegenlage
- Fig. 6: Schematische Seitenansicht eines mit Kappen gefüllten Magazins zur Illustration der Lagerung der Kappe mit einer schrägen Gegenlage
- Fig. 7: Schematische Darstellung einer bevorzugten Ausführungsform eines Mehrfachmagazins mit geöffneten Schiebern (A) und geschlossenen Schiebern (B)

### Detaillierte Beschreibung der Abbildungen

Figur 1 zeigt eine schematische Darstellung einer bevorzugten Ausführungsform einer Kappe **2** für einen POC-Testreifen (nicht gezeigt). In der Fig. 1A wird eine Draufsicht und in Fig. 1B eine Schnittdarstellung gezeigt. Die Kappe **2** umfasst einen Hauptkörper **9** mit einem flüssigkeitsgefüllten Blister **4** sowie zweier seitliche Zangenarme **5** mit Zapfen **7** zur Anbringung der Kappe **2** an dem POC-Teststreifen (nicht gezeigt).

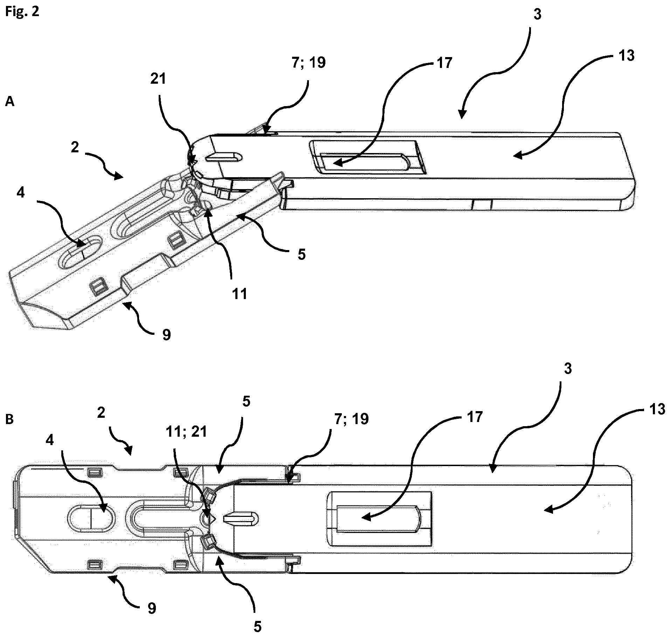

Figur 2 zeigt eine schematische Darstellung einer bevorzugten Ausführungsform eines zusammengesetzten POC-Testsystems aus Kappe **2** und POC-Teststreifen **3.** In der Fig. 2 A wird der geöffnete Zustand gezeigt, während Fig. 2 B den geschlossen Zustand illustriert. Der POC-Teststreifen **3** ist ein immunchromatographischer Testreifen mit funktionellen Bereichen **17,** welche beispielsweise eine Probenregion, eine Markierungsregion, eine Detektionsregion oder eine Absorptionszone umfassen kann. Die funktionellen Bereiche **17** des POC-Teststreifen **3** sind in einem Gehäuse **13** eingefasst, welche eine im Wesentlichen abgeflachte Streifen- bzw. Quaderform aufweist und im vorderen Bereich seitliche Nuten **19** zur Anbringen der Kappen **3** vorsieht. Die Nuten **19** korrespondieren mit den Zapfen **7** der Zangenarme **5** der Kappe **2,** sodass die Kappe **2** kippbar am POC-Teststreifen **3** fixiert werden kann.

Eine Probe (beispielsweise eine Blut-Probe) kann im geöffneten Zustand des POC-Systems (Fig. 2A) in einer dazu eingerichtete Öffnung **21** gegeben werden. Nach Auftragen der Probe ist es in der Regel notwendig eine Pufferlösung oder Laufmittel auf den POC-Teststreifen **3** einzubringen, um die Ausbreitung der Probe über die funktionellen Bereiche **17** des Teststreifens sicherzustellen bzw. Reagenzien für den Nachweis bereitzustellen.

Hierzu wird das POC-System mittels Kippen der Kappe **2** verschlossen (Fig. 2B), wodurch der flüssigkeitsgefüllte Blister **4** über die Öffnung **21** in Fluidkommunikation mit den funktionellen Bereichen **17** gebracht wird. Die Ausstülpung **11** im mittleren Bereich des Hauptkörpers **9** der Kappe **2** gewährleistet einen sichern Verschluss. Die Testreaktion kann mithin gezielt durch eine Beschädigung bzw. Entleeren des flüssigkeitsgefüllten Blisters **4** ausgelöst werden.

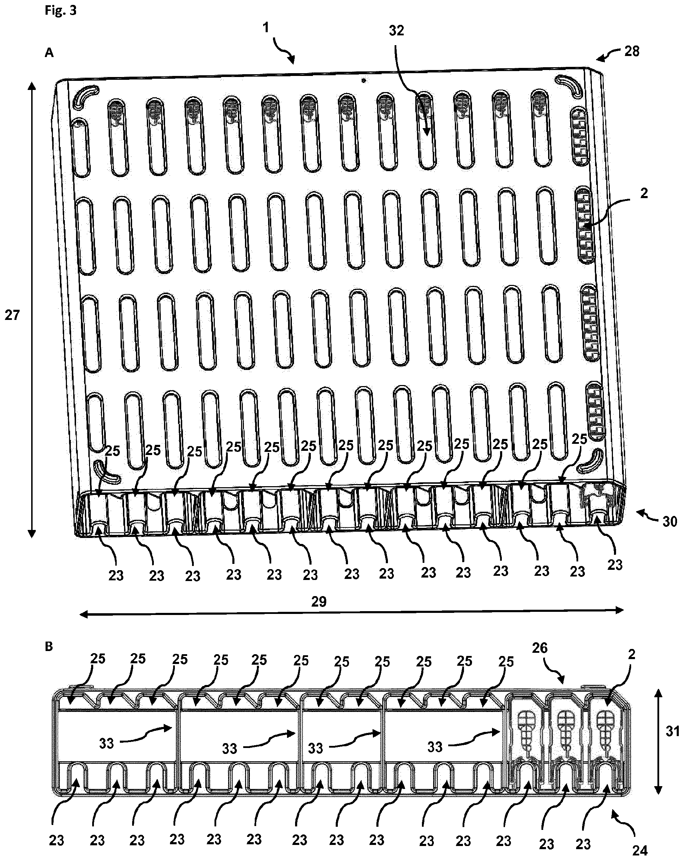

Fig. 3 zeigt eine schematische Darstellung einer bevorzugten Ausführungsform eines Mehrfachmagazins **1,** wobei in Fig. 3A eine Vorderansicht und in Fig. 3 B eine Schnittansicht gezeigt wird. Das Mehrfachmagazin **1** weist eine im Wesentlichen abgeflachte Quaderform auf, mit einer deutlich größere Höhe **27** und Breite **29** im Vergleich zur Tiefe **31.**

In der Vorderansicht der Fig. 3 A des Mehrfachmagazin **1** zeigt das Kopfende **28** nach oben während das Fußende **30** nach unten zeigt. Eine Reihe von vertikalen Fächern des Mehrfachmagazins **1** werden durch Zungen **23** und Ausnehmungen **25** gebildet, welche an gegenüberliegenden Innenflächen des Magazins **1** periodisch angeordnet sind. Die Außenwand des Mehrfachmagazins **1,** an deren Innenfläche die Ausnehmungen **25** vorliegen, wird als Rückwand **26** bezeichnet, während die Außenwand des Mehrfachmagazins **1,** an welcher die Zungen **23** vorliegen, als Vorderwand **24** bezeichnet wird.

Die Zungen **23** erstrecken sich über die gesamte Höhe **27** der Innenfläche der Vorderwand **24** und weisen einen abgerundet rechteckigen Querschnitt auf, dessen Dimensionierung an die Form und Größe der Zangenarme **5** der Kappen **2** angepasst ist. Auf der gegenüberliegenden Innenfläche des Magazins **1** werden die Kappen **2** zudem durch Ausnehmungen **25** stabilisiert, welche kongruent zur Form des Kopfendes der Kappe **2** ausgebildet sind. Die bevorzugt illustrierten Kappen **2** (vgl. auch Fig. 1) weisen eine im Wesentlichen rechteckige Form auf, wobei eine Ecke abgeschrägt vorliegt. Die Ausnehmungen **25** weisen dementsprechend eine im Wesentlichen rechteckige Trapezform auf.

In die durch Zungen **23** und Ausnehmungen **25** gebildeten Fächer lassen sich die Kappen **2** zuverlässig vertikal stapeln. Mittels Trennwände **33** können Kompartimente von beispielsweise zwei oder drei Fächern gebildet werden. Die Trennwände **33** erstrecken sich bevorzugt entlang der gesamten Höhe **27** des Magazins von einer Innenfläche der Rückwand **26** zur gegenüberliegenden Innenfläche der Vorderwand **24.**

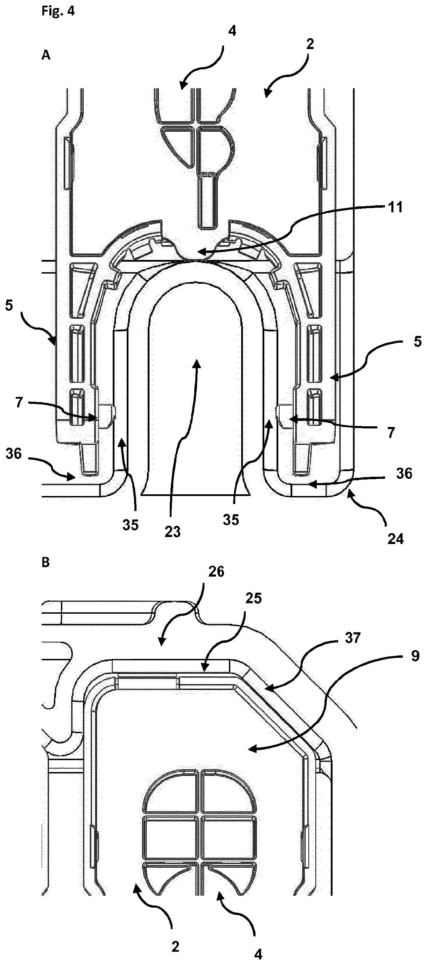

Figur 4 zeigt eine schematische Detailansicht der Fixierung einer Kappe **2** innerhalb der Fächer des Mehrfachmagazins **1.** In der dargestellten Ausführungsform ist die Höhe der Zunge **23** geringfügig, beispielsweise zwischen 0,5 und 3 mm, höher, als die Länge der Zangenarme **5** der Kappe **2,** sodass bei einer Stapelung der Kappen **2** im Mehrfachmagazin **1** die Enden der Zangenarme **5** die Innenfläche der Vorderwand **24** nicht berühren. Wie in Fig. 4A gezeigt, sitzt vielmehr die Kappe **2** mit der Ausstülpung **11** auf der Zunge **23** auf und zwischen dem Ende der Zangenarme **5** und der Innenfläche der Vorderwand **24** des Mehrfachmagazin verbleibt eine Toleranz **36.**

Zudem ist die Breite der Zunge **23,** geringfügig beispielsweise zwischen 1 mm und 2 mm, schmaler, als der Abstand der Zapfen **7** der Zangenarme **5** der Kappen, sodass bei einer Stapelung der Kappen **2** im Mehrfachmagazin die Zapfen **7** der Zangenarme **5** beidseitig mit einer Toleranz **35** geführt werden.

Auch die Tiefe des Mehrfachmagazins und Dimensionierung der Ausnehmungen **25** ist bevorzugt so gewählt, dass eine geringfügige Toleranz **37** zwischen dem Kopfende der Kappe **2** und der Innenfläche der Rückwand **26** verbleibt, sofern die Kappe **2** auf der Zunge **23** aufsitzt (vgl. Fig. 4B). Die Toleranzen erlauben eine sichere Führung und Transport der Kappen **2** mit gleichzeitig hinreichendem Spiel für eine effiziente Be- und Entladung.

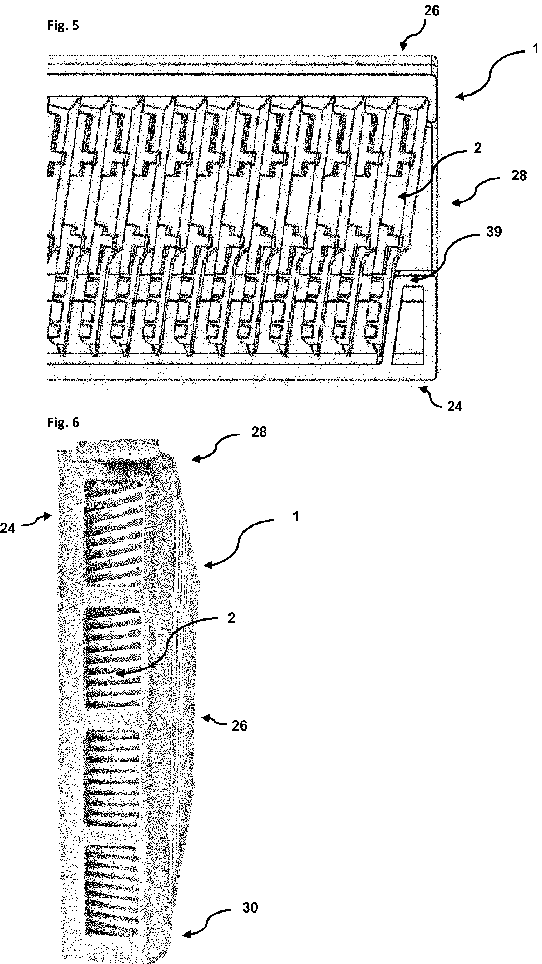

Die Figuren 5 und 6 illustrieren die Funktionsweise der schrägen Gegenlage **39** zur Ausrichtung der Kappen **2** innerhalb der vertikalen Fächer. Die Figur **5** zeigt eine schematische Schnittdarstellung und die Figur 6 eine schematische Seitenansicht eines mit Kappen **2** gefüllten Magazins.

Die schräge Gegenlage **39** definiert bevorzugt eine Steigung von der Innenfläche der Rückwand **26,** an welcher sich die Zungen befinden, zur Vorderwand **24,** an welche sich die Ausnehmungen befinden. In der gezeigten Ausführungsform beträgt der Steigungswinkel 8°. Wie in Fig. 5 gezeigt liegt ein Ende der schrägen Gegenlage **39,** welches zur Vorderwand **24** bzw. den Ausnehmungen orientiert ist, kopfseitiger, als ein Ende der Gegenlage **39,** welches zur Rückwand **26** bzw. den Zungen orientiert ist.

Für eine vertikale Stapelung der Kappen **2** kann das Magazin **1** beispielsweise mit dem Kopfende **28** nach unten aufgestellt werden. Werden die Kappen **2** nunmehr in die Fächer eingebracht, führt die schräge Gegenlage **39** dazu, dass die ersten Kappen **2,** welche sich im Fach unten befinden, innerhalb des Faches eine Neigung aus der Horizontalen aufweisen, welche dem Steigungswinkel der Gegenlage **39** entspricht. In der gezeigten Ausführungsform führt ein Steigungswinkel der Gegenlage **39** von 8° zu einer Schräglage bzw. Neigung von 8° der ersten Kappen **2** gegenüber der Horizontalen. Die folgend eingeführten Kappen **2** werden sukzessiv eine geringere Schräglage aufweisen, da für bevorzugten Kappen **2** die Dicke des Kopfendes höher ist, als das die Dicke der seitlichen Zangenarme. Nach einer bestimmten Anzahl vertikal gestapelten Kappen **2** liegt keine Neigung bzw. Schräglage mehr vor, sodass die letzten Lagen der Kappen **2** eine im Wesentlichen horizontale Ausrichtung erfahren. Es kann auch bevorzugt, sein, dass die letzten Kappen **2** bereits eine geringfügig über die Horizontal hinausgehende Schräglage aufweisen.

In Fig. 6 ist das Mehrfachmagazin **1** mit dem Kopfende **28** nach oben orientiert. Der Verlauf der Ausrichtung der Kappen **2** mit einer initialen Schräglage zu einer horizontalen Ausrichtung zum Fußende **30** hin ist deutlich sichtbar.

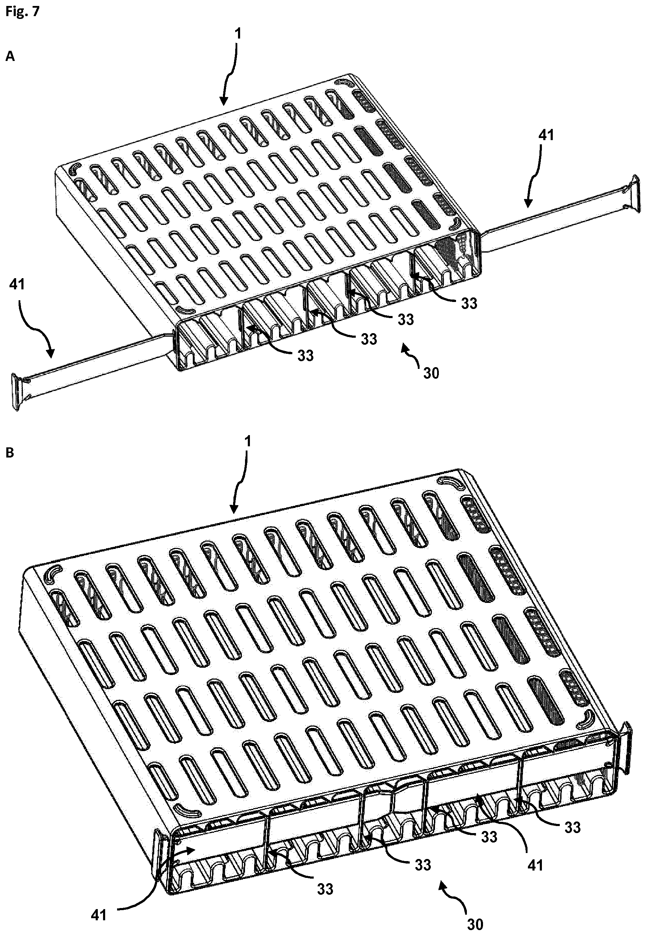

Figur 7 zeigt eine schematische Darstellung einer bevorzugten Ausführungsform eines Mehrfachmagazins **1** mit geöffneten Schiebern **41** (Fig. 7A) und geschlossenen Schiebern **41** (Fig. 7B)

In der gezeigten Ausführungsform weist das Mehrfachmagazin **1** zwei Schieber **41** zum wahlweisen Öffnen bzw. Verschließen des Fußendes **30** des Magazins **1** auf. Die Breite der beiden Schieber **41** ist geringfügig schmaler, als die Tiefe des Mehrfachmagazins **1,** wobei die Gesamtlänge der beiden Schieber **41** bevorzugt der Breite des Mehrfachmagazins **1** entspricht. Vorliegend entspricht die Länge jedes Schiebers **41** ca. einer halben Breite des Mehrfachmagazins, sodass die Schieber **41** symmetrisch das Fußende **30** des Mehrfachmagazins **30** öffnen und verschließen können. Die Schieber **41** werden mittels entsprechender Durchgänge in den Trennwänden **33** geführt.

### BEISPIELE

Das folgende Prüfbeispiel illustriert die Vorteile bevorzugter Fixierungen der Kappen, ohne auf diese beschränkt zu sein.

Die Kappe wird hierzu, wie beschrieben, im unteren Teil (Fußende) durch Auflage auf einzelnen Zungen geführt und durch Ausnehmungen in den Fächern des Magazins gehalten. In Bezug auf den Abstand der Zapfen der Kappen zur Zungen werden unterschiedliche Toleranzen bzw. Freiheitsgrade der Kappe getestet. Hierzu wurde jeweils eine Zungenbreite gewählt, welche um 1mm, 1,5 mm und 2 mm schmaler ist als der Abstand der Zapfen, sodass seitliche Toleranzen bzw. Freiheitsgraden von 0,5 mm, 0, 75 mm und 1 mm gewährleistet werden (vgl. Fig. 4).

Folgende Anforderungen bezogen auf die drei Einsatzfälle (Beladen, Transport und Entladen) werden überprüft:

### Beladen des Magazins

- Handling beim manuellen Beladen (subjektiver Eindruck)
- Spiel der Kappen beim Befüllen = Befüll-Geschwindigkeit
- Bewertung der Kappenausrichtung beim Beladen = Verkanten der Kappen je Reihe
- Kappenausrichtung beim Beladen: Kippeln der Kappen

Zur manuellen Beladung wird eine Beladungshilfe simuliert: Vorgesehen ist die Konstruktion eines Beladungstisches, in den das Magazin eingelegt wird. Anschließend fährt von der Rückseite ein Stempel soweit in das Magazin ein, dass lediglich die ersten zwei Reihen befüllt werden müssen. Dann fährt der Stempel sukzessive in Schritten hinunter, bis der Stempel das Magazin wieder verlassen hat und das Magazin komplett beladen ist.

### Transport des Magazins

- Transportsimulation des Magazins: Vollständige Befüllung und Schütteln/Rütteln des Magazins mit in Ausrichtung liegend mit 200 U/min für 6 h. Anschließend 2 h bei -15°C sowie 1,5 h bei 37 °C.

### Auswertung hinsichtlich Fixierung, Beschädigung und Abrieb

- Temperatursimulation: Lagerung bei -20 °C und 37 °C und Auswertung hinsichtlich verändertem Kappenspiel bzw. Fixierung

### Entladen des Magazins

- Entladungssimulation: "Top-Down" und "Bottom-Up"
- Verhalten jeder Reihe bzgl. Verkippen /Verkanten (speziell erste bzw. letzte Kappe)
- Prüfen in wieweit eine passive (Schwerkraft)-Entladung möglich ist oder bis zu welchem Grad eine aktive Unterstützung notwendig ist
- Prüfen, ob das Spenden einer Kappe durch Verschieben einer geeigneten Aufnahme, beispielsweise mittels eines Spenders mit einer Aussparung, direkt am Magazin möglich ist. Falls ja, ist das Spendeverhalten unauffällig? Wie fällt die Kappe in die Aussparung?

Folgende Ergebnisse wurden erzielt:

| | | **Freiheitsgrad 0,5 mm** | **Freiheitsgrad 0,75 mm** | **Freiheitsgrad 1,0 mm** |
|---|---|---|---|---|
| **1) Beladen:** | | | | |
| a. Handling generell | | unauffällig | unauffällig | unauffällig |
| b. Spiel = Tempo (Zeit) | | 1:41 | 1:52 | 1:35 |
| c. Verkanten (Menge) | | unauffällig | unauffällig | unauffällig |
| d. Kippeln | | Kein oder geringfügiges Kippeln | | |

| **2) Transport:** | | | | |
|---|---|---|---|---|
| a. Kappen-Fixierung | | Gut | Gut | Gut |
| b. Beschädigung | | Keine | Keine | Keine |
| c. Abrieb | | Keiner | Keiner | Keiner |
| d. Kappenspiel (Temp.) | | Nicht feststellbar | Nicht feststellbar | Nicht feststellbar |
| e. Fixierung (Temp.) | | Nicht feststellbar | Nicht feststellbar | Nicht feststellbar |

| **3) Entladen:** | | | | |
|---|---|---|---|---|
| | **Methode** | **Top-Down** | | |
| a. Verkanten (Menge) | | 1 | 0 | 0 |
| b. Unterstützung nötig? | | Unterstützung notwendig (Gewicht ca. 100 g) | | |
| c. Spendeverhalten | | Unauffällig, Fuß zuerst | Unauffällig, Fuß zuerst | unauffällig Fuß zuerst |

| | **Methode** | **Bottom-Up** | | |
|---|---|---|---|---|
| d. Verkanten (Menge) | | 0 | 0 | 0 |
| e. Unterstützung nötig | | N/A | | |
| f. Spendeverhalten | | Leicht erhöhte Reibung | unauffällig | Spiel groß |

Die Prüfergebnisse zeigen, dass die bevorzugte Fixierung der Kappen im Mehrfachmagazin sowohl im Hinblick auf Beladung, Transport, als auch Entladung höchsten Anforderungen genügt. Einerseits wird eine hohe Transportsicherheit gewährleistet, welche Beschädigung oder Abrieb verhindert. Anderseits zeichnet sich das Mehrfachmagazin durch hohe Bedienerfreundlichkeit aus, welche eine zuverlässiges und zügiges Beladen und Entladen der Kappen ermöglicht.

Es wird darauf hingewiesen, dass verschiedene Alternativen zu den beschriebenen Ausführungsformen der Erfindung verwendet werden können, um die Erfindung auszuführen und zu der erfindungsgemäßen Lösung zu gelangen. Das erfindungsgemäße Mehrfachmagazin, der Kit sowie deren Verwendung in den beschriebenen Verfahren beschränken sich in ihren Ausführungen somit nicht auf die vorstehenden bevorzugten Ausführungsformen. Vielmehr ist eine Vielzahl von Ausgestaltungsvarianten denkbar, welche von der dargestellten Lösung abweichen können. Ziel der Ansprüche ist es, den Schutzumfang der Erfindung zu definieren. Der Schutzumfang der Ansprüche ist darauf gerichtet, das erfindungsgemäße Mehrfachmagazin, den Kit und Verfahren unter deren Verwendung sowie äquivalente Ausführungsformen von diesen abzudecken.

### Bezugszeichenliste

- 1: Mehrfachmagazin
- 2: Kappe
- 3: POC-Teststreifen
- 4: Blister
- 5: Zangenarme
- 7: Zapfen
- 9: Hauptkörper
- 11: Ausstülpung
- 13: Gehäuse des POC-Teststreifen
- 17: funktionelle Bereiche des POC Teststreifens
- 19: Nuten zur Aufnahme des Zapfen
- 21: Öffnung für Probe
- 23: Zungen
- 24: Vorderwand des Magazins
- 25: Ausnehmungen
- 26: Rückwand des Magazins
- 27: Höhe des Magazins
- 28: Kopfende des Magazins
- 29: Breite des Magazins
- 30: Fußende des Magazins
- 31: Tiefe des Magazins
- 32: Schlitze in Vorder- bzw. Rückwand des Magazins
- 33: Trennwände
- 35: Toleranz zwischen Zapfen und Zunge
- 36: Toleranz zwischen dem Ende der Zangenarme und der Innenfläche des Mehrfachmagazins
- 37: Toleranz am Kopfende der Kappe
- 39: schräge Gegenlage
- 41: Schieber

## Patentansprüche

1. Mehrfachmagazin (1) zum Transport, zur Aufbewahrung und/oder Vorhaltung einer Vielzahl von Kappen (2) für einen POC-Teststreifen (3), wobei die Kappen (2) jeweils einen Hauptkörper (9) mit einem flüssigkeitsgefüllten Blister (4) und zwei Zangenarme (5) mit Zapfen (7) zur Anbringung der Kappe (2) an den POC-Teststreifen (3) aufweisen,
**dadurch gekennzeichnet, dass**
das Mehrfachmagazin (1) an einer Innenfläche einer Vorderwand (24) eine Reihe von zwei oder mehr sich entlang der Höhe (27) des Mehrfachmagazins erstreckender Zungen (23) zur Führung der Zangenarme (5) der Kappen und auf der gegenüberliegenden Innenfläche einer Rückwand (26) eine Reihe von zwei oder mehr sich entlang der Höhe (27) des Mehrfachmagazins erstreckenden Ausnehmungen (25) zur Führung des Hauptkörpers (9) der Kappen aufweist, sodass durch die gegenüberliegenden Zungen (23) und Ausnehmungen (25) eine Reihe von zwei oder mehr Fächern zur vertikalen Stapelung der Kappen (2) gebildet werden und wobei das Mehrfachmagazin (1) am Kopfende (28) eine schräge Gegenlage (39) aufweist, welche sich über die Breite (29) des Mehrfachmagazins erstreckt und eine Steigung entlang der Richtung der Vorderwand (24) zur Rückwand (26) aufweist.

2. Mehrfachmagazin (1) gemäß dem vorherigen Anspruch
**dadurch gekennzeichnet, dass**
die schräge Gegenlage (39) einen Steigungswinkel von 5° - 15°, bevorzugt von 6° -10° aufweist.

3. Mehrfachmagazin (1) gemäß einem der vorherigen Ansprüche
**dadurch gekennzeichnet, dass**
die Zungenhöhe zwischen 0,5 mm und 3 mm höher ist, als die Länge der Zangenarme (5) der Kappe, sodass bei einer Stapelung der Kappen (2) im Mehrfachmagazin (1) die Enden der Zangenarme (5) das Mehrfachmagazin (1) nicht berühren und/oder die Zungenhöhe zwischen 15 und 25 mm, bevorzugt zwischen 18 und 22 mm beträgt.

4. Mehrfachmagazin (1) gemäß einem der vorherigen Ansprüche
**dadurch gekennzeichnet, dass**
die Zungenbreite zwischen 0,5 mm und 1 mm, bevorzugte zwischen 0,75 mm und 1 mm, schmaler ist, als der Abstand der Zapfen (7) der Zangenarme (5) der Kappen, sodass bei einer Stapelung der Kappen (2) im Mehrfachmagazin (1) die Enden die Zangenarme (5) mittels der Zunge (23) mit einer Toleranz von 0,5 mm bis 1 mm, bevorzugt von 0,75 bis 1 mm, geführt werden und/oder
die Zungenbreite zwischen 8 mm und 12 mm, bevorzugt zwischen 9 mm und 10,5 mm beträgt.

5. Mehrfachmagazin (1) gemäß einem der vorherigen Ansprüche
**dadurch gekennzeichnet, dass**
das Mehrfachmagazin (1) eine Reihe von 5-30, bevorzugt 8-20 Fächern zur vertikalen Stapelung der Kappen (2) aufweist.

6. Mehrfachmagazin (1) gemäß einem der vorherigen Ansprüche
**dadurch gekennzeichnet, dass**
das Mehrfachmagazin (1) in der Höhe (27) derart dimensioniert ist, dass in jedem Fach zwischen 35 und 50, bevorzugt zwischen 40 und 45 Kappen (2) vertikal gestapelt werden können

7. Mehrfachmagazin (1) gemäß einem der vorherigen Ansprüche
**dadurch gekennzeichnet, dass** das Mehrfachmagazin (1) eine Höhe (27) von 20 cm bis 40 cm, bevorzugt 25 bis 30 cm, eine Breite (29) von 15 cm bis 50 cm, bevorzugt von 25 cm bis 40 cm und eine Tiefe (31) von 45 mm bis 75 mm, bevorzugt von 60 - 65 cm aufweist.

8. Mehrfachmagazin (1) gemäß einem der vorherigen Ansprüche
**dadurch gekennzeichnet, dass**
das Mehrfachmagazin (1) zusätzliche Trennwände (33) aufweist, welche bevorzugt jeweils einen Abschnitt von ein, zwei, drei oder mehr Fächern voneinander trennen.

9. Mehrfachmagazin (1) gemäß einem der vorherigen Ansprüche
**dadurch gekennzeichnet, dass**
das Mehrfachmagazin (1) einen Schieber (41), bevorzugt zwei Schieber (41), zum wahlweisen Öffnen bzw. Verschließen des Fußendes (30) des Mehrfachmagazins (1) aufweist.

10. Mehrfachmagazin (1) gemäß einem der vorherigen Ansprüche
**dadurch gekennzeichnet, dass**
das Mehrfachmagazin (1) aus Kunststoff gefertigt ist, beispielsweise aus Acrylnitril-Butadien-Styrol (ABS) oder Polyethylen (PE).

11. Verwendung eines Mehrfachmagazins (1) gemäß einem der vorherigen Ansprüche zur Aufbewahrung, zum Transport und/oder Vorhaltung einer Vielzahl von Kappen (2) für einen POC-Teststreifen (3).

12. Kit umfassend
a. ein Mehrfachmagazin (1) gemäß einem der vorherigen Ansprüche 1-10
b. Eine Vielzahl von Kappen (2) für einen POC-Teststreifen (3), wobei die Kappen (2) jeweils einen Hauptkörper (9) mit einem flüssigkeitsgefüllten Blister (4) und zwei Zangenarme (5) mit Zapfen (7) zur Anbringung der Kappe (2) an dem POC-Teststreifen (3) aufweisen,
wobei die Vielzahl von Kappen (2) bevorzugt in den Fächern des Mehrfachmagazins (1) vertikal gestapelt vorliegen.

13. Verfahren zur Montage einer Vielzahl von POC-Testsystemen umfassend die Schritte
a. Bereitstellung eines Mehrfachmagazins (1) gemäß einem der vorherigen Patentansprüche 1-10, wobei innerhalb des Mehrfachmagazins (1) eine Vielzahl von Kappen (2) für einen POC-Teststreifen (3) vorliegen, welchen einen Hauptkörper (9) mit einem flüssigkeitsgefüllten Blister (4) und zwei Zangenarme (5) mit Zapfen (7) zur Anbringung an einen POC-Teststreifen (3) umfassen
b. Bereitstellung einer Vielzahl von POC-Teststreifen (3) mit beidseitigen Nuten (19) zur Aufnahme der Zapfen (7) der Kappen
c. Individuelle Entladung der Kappen (2) aus dem Mehrfachmagazin (1) auf einen Montagetisch und/oder ein Transportband
d. Aufsetzen der Kappe (2) auf den POC-Teststreifen (3), wobei die Zapfen (7) der Zangenarme (5) in die beidseitigen Nuten (19) des POC-Teststreifens (3) einrasten.

14. Verfahren zur Montage einer Vielzahl von POC-Testsystemen gemäß dem vorherigen Anspruch
**dadurch gekennzeichnet, dass**
die individuelle Entladung der Kappen (2) aus dem Mehrfachmagazin (1) ausschließlich aufgrund der Schwerkraft erfolgt und/oder
die individuelle Entladung der Kappen (2) aus dem Mehrfachmagazin (1) durch Verwendung eines Kolbens unterstützt wird, welcher die in einem Fach des Mehrfachmagazins (1) vertikal gestapelten Kappen (2) ausstößt.

15. Verfahren zur Montage einer Vielzahl von POC-Testsystemen gemäß einem der vorherigen Ansprüche 13 oder 14
**dadurch gekennzeichnet, dass**
zur Entladung der Kappen (2) das Mehrfachmagazin (1) mit der Fußseite (30) auf einen Abstandshalter gestellt wird und eine individuelle Entnahme der Kappen (2) mittels eines Schiebers erfolgt, welcher eine Aussparung zur Aufnahme für die Kappen (2) aufweist.

## Claims

1. Multiple magazine (1) for transporting, storing, and/or providing a plurality of caps (2) for a POC test strip (3), the caps (2) each having a main body (9), which has a liquid-filled blister (4), and two pincer arms (5), which have pins (7) for attaching the cap (2) to the POC test strips (3),
**characterized in that**
the multiple magazine (1) has, on an inner surface of a front wall (24), a row of two or more tongues (23) that extend along the height (27) of the multiple magazine to guide the pincer arms (5) of the caps, and has, on the opposite inner surface of a rear wall (26), a row of two or more recesses (25) that extend along the height (27) of the multiple magazine to guide the main body (9) of the caps, so that a row of two or more compartments for vertically stacking the caps (2) is formed by the opposite tongues (23) and recesses (25), the multiple magazine (1) having, at the top end (28), an inclined counter-bearing (39) which extends across the width (29) of the multiple magazine and has an incline in the direction from the front wall (24) to the rear wall (26).

2. Multiple magazine (1) according to the previous claim,
**characterized in that**
the sloped counter-bearing (39) has a incline angle of 5°-15°, preferably 6°-10°.

3. Multiple magazine (1) according to either of the preceding claims,
**characterized in that**
the tongue height is between 0.5 mm and 3 mm higher than the length of the pincer arms (5) of the cap, so that when the caps (2) are stacked in the multiple magazine (1), the ends of the pincer arms (5) do not touch the multiple magazine (1), and/or the tongue height is between 15 and 25 mm, preferably between 18 and 22 mm.

4. Multiple magazine (1) according to any of the preceding claims,
**characterized in that**
the tongue width is between 0.5 mm and 1 mm, preferably between 0.75 mm and 1 mm, narrower than the distance between the pins (7) of the pincer arms (5) of the caps, so that when the caps (2) are stacked in the multiple magazine (1), the ends of the pincer arms (5) are guided by means of the tongue (23) with a tolerance of 0.5 mm to 1 mm, preferably 0.75 to 1 mm, and/or
the tongue width is between 8 mm and 12 mm, preferably between 9 mm and 10.5 mm.

5. Multiple magazine (1) according to any of the preceding claims,
**characterized in that**
the multiple magazine (1) has a row of 5-30, preferably 8-20 compartments for vertically stacking the caps (2).

6. Multiple magazine (1) according to any of the preceding claims,
**characterized in that**
the height (27) of the multiple magazine (1) is dimensioned in such a way that between 35 and 50, preferably between 40 and 45, caps (2) can be vertically stacked in each compartment.

7. Multiple magazine (1) according to any of the preceding claims,
**characterized in that** the multiple magazine (1) has a height (27) of from 20 cm to 40 cm, preferably from 25 to 30 cm, a width (29) of from 15 cm to 50 cm, preferably from 25 cm to 40 cm, and a depth (31) of from 45 mm to 75 mm, preferably from 60 to 65 cm.

8. Multiple magazine (1) according to any of the preceding claims,
**characterized in that**
the multiple magazine (1) has additional partitions (33), each of which preferably separates a portion of one, two, three or more compartments from one another.

9. Multiple magazine (1) according to any of the preceding claims,
**characterized in that**
the multiple magazine (1) has a slider (41), preferably two sliders (41), for selectively opening or closing the bottom end (30) of the multiple magazine (1).

10. Multiple magazine (1) according to any of the preceding claims,
**characterized in that**
the multiple magazine (1) is made of plastic, for example acrylonitrile butadiene styrene (ABS) or polyethylene (PE).

11. Use of a multiple magazine (1) according to any of the preceding claims for storing, transporting and/or providing a plurality of caps (2) for a POC test strip (3).

12. Kit, comprising
a. a multiple magazine (1) according to any of the preceding claims 1-10,
b. a plurality of caps (2) for a POC test strip (3), wherein each cap (2) has a main body (9), which has a liquid-filled blister (4), and two pincer arms (5), which have pins (7) for attaching the cap (2) to the POC test strip (3),
wherein the plurality of caps (2) are preferably stacked vertically in the compartments of the multiple magazine (1).

13. Method for assembling a plurality of POC test systems, comprising the steps of
a. providing a multiple magazine (1) according to any of the preceding claims 1-10, wherein a plurality of caps (2) for a POC test strip (3) are provided within the multiple magazine (1), which caps have a main body (9), which has a liquid-filled blister (4), and two pincer arms (5), which have pins (7) for attachment to a POC test strip (3)
b. providing a plurality of POC test strips (3) which have grooves (19) on both sides for receiving the pins (7) of the caps
c. individually discharging the caps (2) from the multiple magazine (1) onto an assembly table and/or a conveyor belt
d. placing the cap (2) on the POC test strip (3), wherein the pins (7) of the pincer arms (5) snap into the grooves (19) on both sides of the POC test strip (3).

14. Method for assembling a plurality of POC test systems according to the preceding claim,
**characterized in that**
the individual discharging of the caps (2) from the multiple magazine (1) takes place exclusively due to gravity and/or
the individual discharging of the caps (2) from the multiple magazine (1) is assisted by the use of a piston which ejects the caps (2) vertically stacked in a compartment of the multiple magazine (1).

15. Method for assembling a plurality of POC test systems according to either of the preceding claims 13 and 14,
**characterized in that**
in order to discharge the caps (2), the bottom face (30) of the multiple magazine (1) is placed on a spacer and the caps (2) are individually removed by means of a slider which has a recess for receiving the caps (2).

## Revendications

1. Magasin multiple (1) permettant de transporter, de stocker et/ou de fournir une pluralité de capuchons (2) pour une bandelette de test POC (3), les capuchons (2) présentant respectivement un corps principal (9) comportant un blister rempli de liquide (4) et deux bras de pince (5) comportant des chevilles (7) et permettant de fixer le capuchon (2) à la bandelette de test POC (3),
**caractérisé en ce que**
le magasin multiple (1) présente, sur une surface interne d'une paroi avant (24), une série de deux languettes (23) ou plus s'étendant le long de la hauteur (27) du magasin multiple et permettant de guider les bras de pince (5) des capuchons et, sur la surface interne opposée d'une paroi arrière (26), une série de deux évidements (25) ou plus s'étendant le long de la hauteur (27) du magasin multiple et permettant de guider le corps principal (9) des capuchons, de sorte que les languettes (23) et les évidements (25) opposés forment une série de deux compartiments ou plus pour l'empilement vertical des capuchons (2) et le magasin multiple (1) présentant, au niveau de l'extrémité supérieure (28), un contre-support incliné (39), lequel s'étend sur la largeur (29) du magasin multiple et présente une pente le long de la direction de la paroi avant (24) vers la paroi arrière (26).

2. Magasin multiple (1) selon la revendication précédente
**caractérisé en ce que**
le contre-support incliné (39) présente un angle d'inclinaison de 5° à 15°, de préférence de 6° à 10°.

3. Magasin multiple (1) selon l'une des revendications précédentes
**caractérisé en ce que**
la hauteur de languette est supérieure de 0,5 mm à 3 mm à la longueur des bras de pince (5) du capuchon, de sorte que lorsque les capuchons (2) sont empilés dans le magasin multiple (1), les extrémités des bras de pince (5) ne touchent pas le magasin multiple (1) et/ou
la hauteur de languette est comprise entre 15 et 25 mm, de préférence entre 18 et 22 mm.

4. Magasin multiple (1) selon l'une des revendications précédentes
**caractérisé en ce que**
la largeur de languette est inférieure de 0,5 mm à 1 mm, de préférence de 0,75 mm à 1 mm, à la distance entre les chevilles (7) des bras de pince (5) des capuchons, de sorte que lorsque les capuchons (2) sont empilés dans le magasin multiple (1), les extrémités des bras de pince (5) sont guidées au moyen de la languette (23) avec une tolérance de 0,5 mm à 1 mm, de préférence de 0,75 à 1 mm, et/ou la largeur de languette est comprise entre 8 mm et 12 mm, de préférence entre 9 mm et 10,5 mm.

5. Magasin multiple (1) selon l'une des revendications précédentes
**caractérisé en ce que**
le magasin multiple (1) présente une rangée de 5 à 30, de préférence de 8 à 20, compartiments pour l'empilement vertical des capuchons (2).

6. Magasin multiple (1) selon l'une des revendications précédentes
**caractérisé en ce que**
le magasin multiple (1) est dimensionné en hauteur (27) de telle manière qu'entre 35 et 50, de préférence entre 40 et 45, capuchons (2) peuvent être empilés verticalement dans chaque compartiment.

7. Magasin multiple (1) selon l'une des revendications précédentes
**caractérisé en ce que** le magasin multiple (1) présente une hauteur (27) de 20 cm à 40 cm, de préférence de 25 à 30 cm, une largeur (29) de 15 cm à 50 cm, de préférence de 25 cm à 40 cm et une profondeur (31) de 45 mm à 75 mm, de préférence de 60 à 65 cm.

8. Magasin multiple (1) selon l'une des revendications précédentes
**caractérisé en ce que**
le magasin multiple (1) présente des cloisons supplémentaires (33), lesquelles séparent de préférence respectivement une section d'un, de deux, de trois compartiments ou plus les unes des autres.

9. Magasin multiple (1) selon l'une des revendications précédentes
**caractérisé en ce que**
le magasin multiple (1) présente un coulisseau (41), de préférence deux coulisseaux (41), permettant d'ouvrir ou de fermer sélectivement l'extrémité inférieure (30) du magasin multiple (1).

10. Magasin multiple (1) selon l'une des revendications précédentes
**caractérisé en ce que**
le magasin multiple (1) est fabriqué en matière plastique, par exemple en acrylonitrile butadiène styrène (ABS) ou en polyéthylène (PE).

11. Utilisation d'un magasin multiple (1) selon l'une des revendications précédentes permettant de stocker, de transporter et/ou de fournir une pluralité de capuchons (2) pour une bandelette de test POC (3).

12. Kit comprenant
a. un magasin multiple (1) selon l'une des revendications précédentes 1 à 10
b. une pluralité de capuchons (2) pour une bandelette de test POC (3), les capuchons (2) présentant respectivement un corps principal (9) comportant un blister rempli de liquide (4) et deux bras de pince (5) comportant des chevilles (7) et permettant de fixer le capuchon (2) à la bandelette de test POC (3),
dans lequel la pluralité de capuchons (2) se trouvent de préférence empilés verticalement dans les compartiments du magasin multiple (1).

13. Procédé permettant le montage d'une pluralité de systèmes de test POC comprenant les étapes
a. de fourniture d'un magasin multiple (1) selon l'une des revendications précédentes 1 à 10, une pluralite de capuchons (2) pour une bandelette de test POC (3) se trouvant à l'intérieur du magasin multiple (1), lesquels comprenant un corps principal (9) comportant un blister rempli de liquide (4) et deux bras de pince (5) comportant des chevilles (7) et permettant la fixation à une bandelette de test POC (3)
b. de fourniture d'une pluralité de bandelettes de test POC (3) comportant des rainures (19) des deux côtés pour la réception des chevilles (7) des capuchons
c. de déchargement individuel des capuchons (2) du magasin multiple (1) sur une table de montage et/ou un tapis roulant
d. de pose du capuchon (2) sur la bandelette de test POC (3), les chevilles (7) des bras de pince (5) s'emboîtant dans les rainures (19) des deux côtés de la bandelette de test POC (3).

14. Procédé permettant le montage d'une pluralité de systèmes de test POC selon la revendication précédente
**caractérisé en ce que**
le déchargement individuel des capuchons (2) du magasin multiple (1) s'effectue exclusivement par gravité et/ou
le déchargement individuel des capuchons (2) du magasin multiple (1) est supporté par l'utilisation d'un piston qui éjecte les capuchons (2) empilés verticalement dans un compartiment du magasin multiple (1).

15. Procédé permettant le montage d'une pluralité de systèmes de test POC selon l'une des revendications précédentes 13 ou 14
**caractérisé en ce que**
pour le déchargement des capuchons (2), le magasin multiple (1) est placé avec l'extrémité inférieure (30) sur une entretoise et les capuchons (2) sont retirés individuellement au moyen d'un coulisseau qui présente une encoche permettant de recevoir les capuchons (2).
